# EUROPEAN PATENT APPLICATION

(11) **EP 4 512 463 A2**
(43) Date of publication of application: **26.02.2025**
(21) Application number: 25151703.3
(22) Date of filing: 27.11.2020
(51) Int. Cl.: A61N 1/05

(54) **ELECTRODE DEVICES FOR NEUROMODULATION**

(30) Priority: 27.11.2019 US 201962941311 P
(62) Divisional of application: 20834106.5
(71) Applicant: Galvani Bioelectronics Limited, Stevenage SG1 2NY (GB)
(72) Inventor: OUCHOUCHE, Sebastien, Brentford, TW8 9GS (GB); PETERSON, David Karl Lee, Collegeville, 19426 (US); IRWIN, Eric, Collegeville, 19426 (US); COATNEY, Robert W., Collegeville, 19426 (US); YAFFE, Benjamin, Brentford, TW8 9GS (GB); HANSEN, Morten, Brentford, TW8 9GS (GB); ZAIDI, Faisal, Collegeville, 19426 (US); BURGE, Thomas, Brentford, TW8 9GS (GB); KO, Pey-Jiun, Brentford, TW8 9GS (GB); AU, Cindy, Brentford, TW8 9GS (GB); MATTEUCCI, Paul, Brentford, TW8 9GS (GB); PALMER, Mark Gregory, Brentford, TW8 9GS (GB); HUNSBERGER, Gerald Edwin, Brentford, TW8 9GS (GB)
(74) Representative: Carpmaels & Ransford LLP

(57) **Abstract**

In embodiments a neural interface comprising at least one C-ring portion can be used to apply a pressure in a rangej of 0 mmHg to 30 mmHg to a target tissue arranged within the C-ring portion and comprising at least one electrode arranged on the at least one C-ring portion.

## Description

### TECHNICAL FIELD

The present disclosure relates generally to neuromodulation and more particularly to embodiments of extravascular and intravascular devices comprising electrodes for neuromodulation.

### BACKGROUND

Electrical devices of various shapes and sizes including one or more electrodes have been used for neurostimulation/neuromodulation of target anatomy.

Conventional designs lack radial flexibility and self-sizing capabilities. If the target vessel is excessively compressed by the device, nerve damage may result from the decreased blood flow and constricted nerve fibers. Temporary swelling of the target vessel caused by the trauma of the positioning of the device can exacerbate such nerve damage. In contrast, loose fitting devices can result in poor electrical contact and low treatment efficiency, which can further degrade over times as a result of ingrowth of connective tissue between the target vessel and the device.

### SUMMARY

In an embodiment, a neural interface comprises at least one C-ring portion for applying a radial pressure in a range of 1 mmHg to 30 mmHg to a target tissue arranged within the C-ring portion and comprising at least one electrode arranged on the at least one C-ring portion.

The neural interface can further include a lead body comprising a conductor connectable to an implantable pulse generator, wherein the at least one electrode is electrically coupled to the conductor. The C-ring portion can apply a radial pressure based upon rigidity of an insulating material that makes up a body of the C-ring portion, thickness of an insulating material that makes up the body of the C-ring portion, rigidity of the at least one electrode, a size and shape of the at least one electrode, a quantity of electrodes, a proportion of electrode compared to the insulating material of the C-ring portion, a gap size between two electrodes of the at least one electrode, properties of the interconnect between different electrodes of the at least one electrode, thickness of the c-ring material, and a diameter of the neural interface. The C-ring portion(s) can have an inner diameter and a cross-sectional thickness, with a ratio of the inner diameter to the cross-sectional thickness being in a range of 5:1 to 6:1. The electrode can include an electrode contact on an electrode flange, the electrode flange mechanically coupling the electrode to the C-ring portion and comprising a plurality of perforations. The electrode flange can be rectangular with rounded corners. The electrode flange can include a curved under edge. The plurality of perforations can include at least one perforation on a first side of the electrode flange and at least one perforation on a second opposing side of the electrode flange. The first side of the electrode flange and the second opposing side of the electrode flange can be longer than a third side and a fourth side of the electrode flange. The plurality of perforations can be rectangular with rounded corners. The lead body can include at least one strain relieving undulating section.

The neural device can include a spinal portion having a first end and a second end, a circumference of the first end of the spinal portion tapering from a maximum circumference to a minimum circumference, and the lead body can be coupled to the first end of the spinal portion and extending at least partially into the spinal portion. The spinal portion can have a substantially circular cross-section, and the second end of the spinal portion has an angled surface such that a plane parallel to the substantially circular cross-section is at an angle of greater than 0 degrees and less than 90 degrees with respect to a plane defined by the angled surface. The maximum circumference of the first end of the spinal portion can be found proximate the at least three C-ring portions, and the minimum circumference of the first end of the spinal portion occurs where the spinal portion terminates on the lead body. The distance between the maximum circumference and the minimum circumference can be in a range of 2 mm to 5 mm.

The neural interface can include at least two further C-ring portions, each C-ring portion having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion. The first C-ring portion and the third C-ring portion can be coupled to the spinal portion to move together and relative to the second C-ring portion, and wherein the first C-ring portion and the third C-ring portion extend from the spinal portion in a direction opposing a direction of the second C-ring portion. At least one of the C-ring portions can have a first thickness at the first end, a second thickness at the second end, and a third thickness at a point between the first end and the second end, such that the third thickness is greater than the first thickness and the second thickness. A thickness of any of the C-ring portions can gradually increase between the first end and the point between the first end and the second end. A plurality of electrodes can be arranged on at least one of the at least three C-ring portions, wherein adjacent electrodes on the same C-ring portion are electrically coupled by an inter-electrode coil.

The neural interface can include at least one anchoring tab coupled to the lead body. The anchoring tab can include a coated mesh, optionally wherein the mesh is coated with a material that fills the mesh. The C-ring portion can be provided at a first end of the lead body and a connector to an implantable pulse generator (IPG) is provided at a second of the lead body, further wherein the anchoring tab is provided between the first end and the second end of the lead body. The anchoring tab can be provided between the first end of the lead body and a middle section of the lead body situated half-way between the first end and the second end of the lead body, further wherein a ratio of a distance between the first end of the lead body and the anchoring tab and a distance between the second end of the lead body and the anchoring tab is between 1:1 and 1:50, optionally 1:2, 1:3, 1:4 or 1:5. The anchoring tab can be moveable along the lead body. The lead body can have increased flexibility in a portion closer to the C-ring portion compared to a portion of the lead body further away from the C-ring portion.

According to another embodiment, a system includes the neural interface as described above, as well as a deployment tool being removably coupleable to the neural interface for deployment of the neural interface. The deployment tool can include a first area configured to be positioned near the neural interface, and a connector for releasably coupling the first area to the neural interface, anchored to the first area. The deployment tool can have a planar shape or a triangular shape, in embodiments. The deployment tool can also include a second area and a central area between the first area and the second area. The first area can be wider than the second area. A cut through the deployment tool can cut through the connector and releases the coupling between the deployment tool and the neural interface for at least the first area to move away from the neural interface device. The deployment tool can further include at least one passage extending from the first area to the second area through the central area, each passage including a first opening in the first area and a second opening in the second area. The connector can be a suture thread for passing through the at least one passage from the second opening to the first opening and for holding the first area near the implantable device, and anchored to the first area. The deployment tool can further include a cuttable portion extending across the at least one passage and configured to release at least one portion of the connector within the at least one passage when the cuttable portion is cut through, wherein the release of the at least one portion of the suture thread enables the first area to move away from the implantable device.

The connector can include a first portion that passes through the at least one passage from the second opening to the first opening, wherein the connector includes a second portion that is removably attached to the implantable device, wherein the connector includes a third portion that passes through the at least one passage from the first opening to the second opening, and wherein the first portion is connected to the second portion and the second portion is connected to the third portion. Systems can include both a first passage and a second passage, wherein the first portion passes through the first passage, the third portion passes through the second passage. The first area and the second area can include rounded edges. The cuttable portion can be a depressed area in the central area that extends across at least the first passage and the second passage. The depressed area in the central area can extend only across a portion of width of the central area so that at least a portion of the central area is not cut into two pieces when the depressed area is cut through to release the connector. The depressed area can extend across a whole width of the central area so that the central area is cut into two pieces when the depressed area is cut through to release the connector. The central area can include a series of alternating lateral ridges and lateral valleys that extend across a width of the central area, for providing longitudinal flexibility that enables the deployment tool to be rolled up while providing lateral stiffness when the deployment tool is unrolled. The first area and the second area can include the alternating lateral ridges and lateral valleys that extend across a width of the first area and a width of the second area. The passage can be formed by a tunnel through each lateral ridge and a tube across each lateral valley. The cuttable portion can be a lateral valley. The connector can be anchored to the first area by being molded into the first area. The connector can be anchored to the first area by adhesive. The first area, second area, and central area can be molded from silicone. The second area can be tapered toward the second opening. The tapered second area can include a gripping point for manipulation, and the gripping point can include an opening. The deployment tool can include a first surface and a second surface opposite the first surface, the first surface providing an indication of the location of the cuttable portion, the second surface including a plurality of longitudinal grooves along a length of the deployment tool for reduced contact. The second area and the central area can be tapered, and a first portion of the plurality of longitudinal grooves can extend from the first area to the second area through the central area and a second portion of the plurality of longitudinal grooves extend from the first area to the central area. The second area can taper in thickness from an edge of the second area towards the central area. The thickness can increase from the edge of the second area towards the central area. The second area can include a rounded edge.

The neural interface can be a cuff that includes a spine and at least two curved arms extending from the spine and comprising electrodes, wherein each open end of the curved arm is removably coupled to the deployment tool. The neural interface can include a first arm for being moved in a first direction and one or more second arms for being moved in a second direction substantially opposite the first direction, and wherein the second portion of the connector is removably attached to the one or more second arms. The second arms can include two arms positioned on opposite sides of the first arm, one arm among the two arms aligned with the first opening of the first passage and the other arm among the two arms aligned with the first opening of the second passage. The second arms can include corresponding eyelets, and the second portion of the connector can be removably attached to the cuff by passing through the first eyelet and the second eyelet so as to hold the first area near the cuff until at least one of the first portion or the third portion is cut through at the cuttable portion so that the second portion of the connector can be pulled away from the cuff. The thickness of the central area of the tab can be equal or larger than a thickness of the neural interface. The second arms can have an arm height in a direction perpendicular to both a width and length of the tab, wherein the central area has a height that runs substantially parallel to the arm height, and wherein the height of the central area is greater than the arm height. The width of the first area of the tab can be equal or larger than a width of the neural interface. The cuff can have a width measured from an outer side of the one arm to an outer side of the other arm and that runs substantially parallel to the width of the first area, and wherein the width of the first area is greater than a width of the cuff. The deployment tool can be configurable as a measurement tool for measuring a fit of the neural interface to a target. A measurement of a fit can be determined based on a distance between the ridges or grooves or valleys of the deployment tool. The measurement of a fit can be determined based on a distance between a first portion of the deployment tool and a second portion of the deployment tool. The deployment tool can be configured to function as a blunt dissection tool. A thickness of the deployment tool can be larger than a thickness of a C-ring-portion of the neural interface. A width of the deployment tool can be larger than a width of the neural interface.

The deployment tool can be positioned within the C-ring portion of the neural interface. The deployment tool can be rolled at least partly in the neural interface, for example in the C-ring portion. The deployment tool can thus be configured to protect electrodes in the C-ring portion until deployment of the neural interface.

The systems described above may further include a lead cap device having a first end and a second end and comprising a body defining an internal cavity that extends from the first end toward the second end, and a suture loop coupled to the second end, the lead cap device configured to removably receive a portion of the lead body in the internal cavity. An IPG connector portion of the lead body can be removably received in the internal cavity of the lead cap device, further wherein the lead cap comprises a set screw block arranged in the body such that a setscrew intersects with the internal cavity and is configured to secure the portion of the lead body in the internal cavity by the setscrew. In some embodiments, the system comprises a neural interface as disclosed above and a lead cap device (i.e. without the deployment tool).

A system as described above can include inner diameters of the neural interface devices that differ whilst a total electrode area of each neural interface device is substantially equal. The electrode of a larger inner diameter neural interface device can have a smaller width and a larger length than an electrode of a smaller inner diameter neural interface device. A plurality of electrodes can be electrically connected in parallel. The conductor can include a single continuous coil electrically coupled to a plurality of electrodes on one of the C-ring portions. The single continuous coil can include a conductive bushing corresponding to each electrode. The conductive bushing can be crimped for mechanical and electrical connection with the single continuous coil, further wherein each crimped bushing is configured to be welded to each corresponding electrode such that the coil is electrically connected to the electrode. The electrode can have an inbuilt sleeve for accommodating the single continuous coil. A ratio of the gap between interconnecting electrodes to the interconnector can be between 1:2 - 1:3.

In an embodiment, a system comprises a neural interface as disclosed herein, including that of the preceding paragraph; and a deployment tool being removably coupleable to the neural interface for deployment of the neural interface.

In an embodiment, an implantable system comprises a neural interface as disclosed herein, including that of any preceding paragraph; and an anchoring tab configured to be secured to a right crus of the diaphragm.

The anchoring tab may be as disclosed herein, including that of any preceding paragraph describing the anchoring tab. The above summary is not intended to describe each illustrated embodiment or every implementation of the subject matter hereof. The figures and the detailed description that follow more particularly exemplify various embodiments.

### BRIEF DESCRIPTION OF THE DRAWINGS

The subject matter of this disclosure may be more completely understood in consideration of the following detailed description of various embodiments in connection with the accompanying figures, in which:
FIG. 1 is a perspective view of a first side of an embodiment of a bipolar electrode device including a flexible semi-helical structure for holding the electrodes and positioning the device.
FIG. 2A is a perspective view of an opposite side of the embodiment of FIG. 1.
FIG. 2B is a perspective view of an embodiment of a multipolar electrode device including a flexible semi-helical structure similar to FIG. 1 and FIG. 2A.
FIG. 3 is a perspective view of a first side of an embodiment of a tripolar electrode device including a flexible structure.
FIG. 4A is a perspective view of an opposite side of the embodiment of FIG. 3.
FIG. 4B is a perspective view of an embodiment of a bipolar electrode device including a flexible structure similar to FIG. 3 and FIG. 4A.
FIG. 5 is a perspective view of an embodiment of an extravascular Venus FlyTrap electrode device.
FIG. 6 is a perspective view of an embodiment of an extravascular Venus FlyTrap electrode device.
FIG. 7 is a perspective view of an embodiment of an intravascular Venus FlyTrap electrode device.
FIG. 8A is a perspective view of an embodiment of an extravascular bipolar electrode device including a flexible structure similar to FIG. 4B.
FIG. 8B is a perspective view of components of the embodiment of FIG. 8A.
FIG. 8C-1 is a perspective view of an embodiment of a deployment tool.

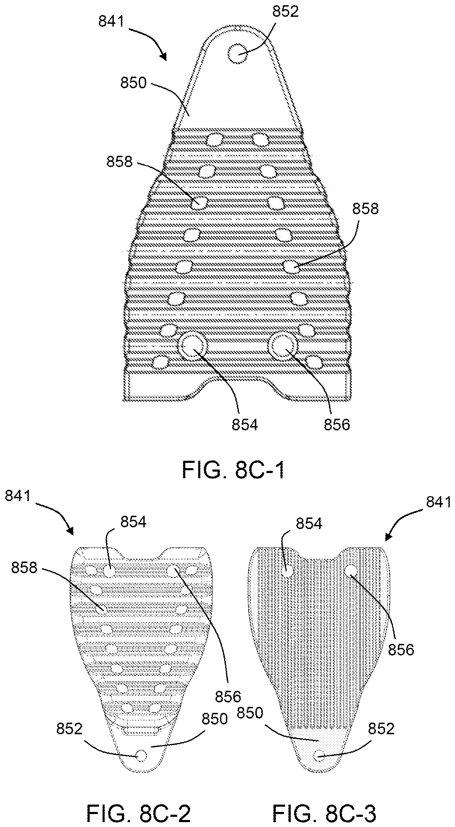

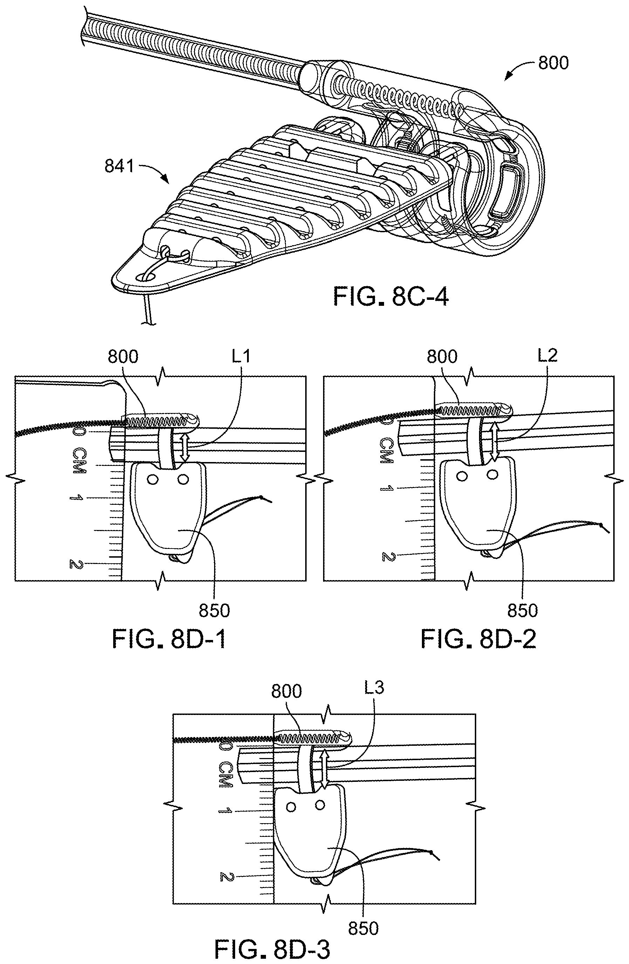

FIG. 8C-2 is a perspective view of another embodiment of a deployment tool.
FIG. 8C-3 is a perspective view of yet another embodiment of a deployment tool.
FIG. 8C-4 is a perspective view of an embodiment of a deployment tool releasably attached to a neural interface device.
FIG. 8D-1 is another view of the deployment tool of FIG. 8C.
FIG. 8D-2 is another view of the deployment tool of FIG. 8C.
FIG. 8D-3 is another view of the deployment tool of FIG. 8C.

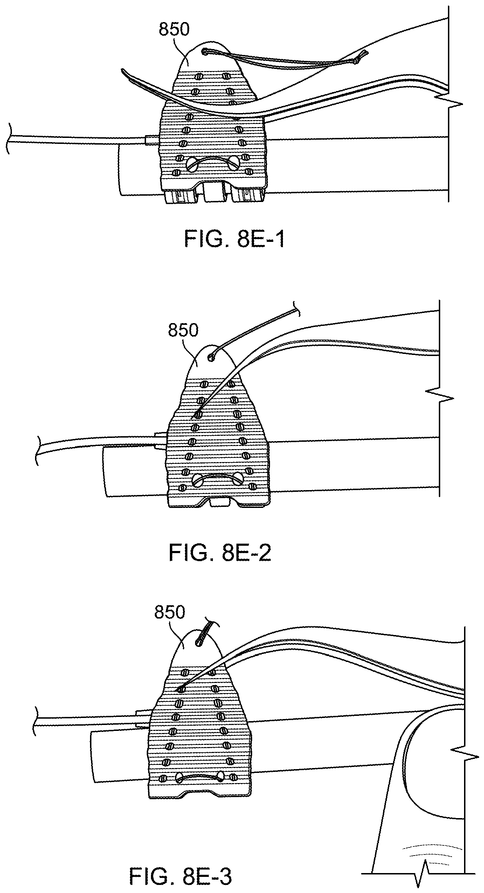

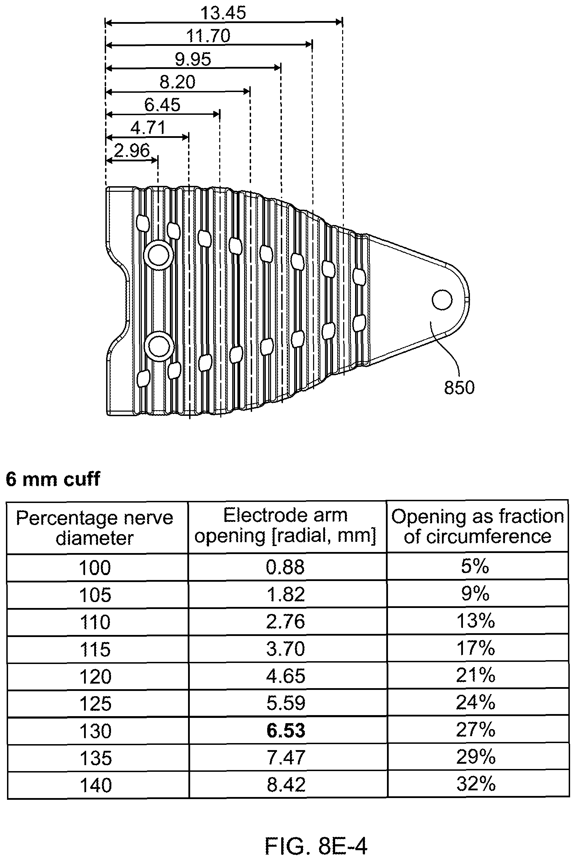

FIG. 8E-1 is another view of the deployment tool of FIG. 8C.
FIG. 8E-2 is another view of the deployment tool of FIG. 8C.
FIG. 8E-3 is another view of the deployment tool of FIG. 8C.

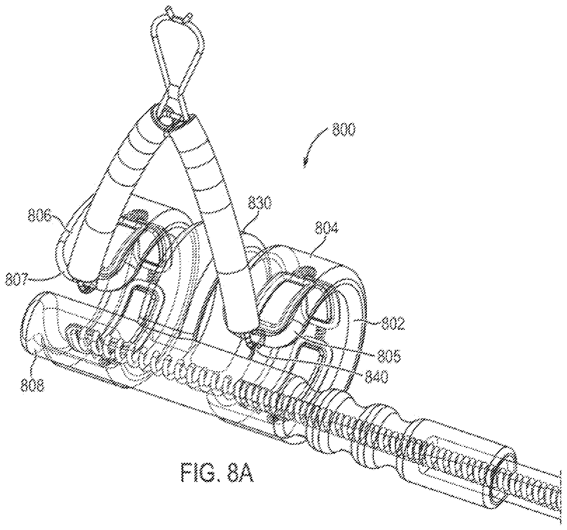

FIG. 8E-4 is another view of the deployment tool of FIG. 8C and a corresponding table of dimensions.

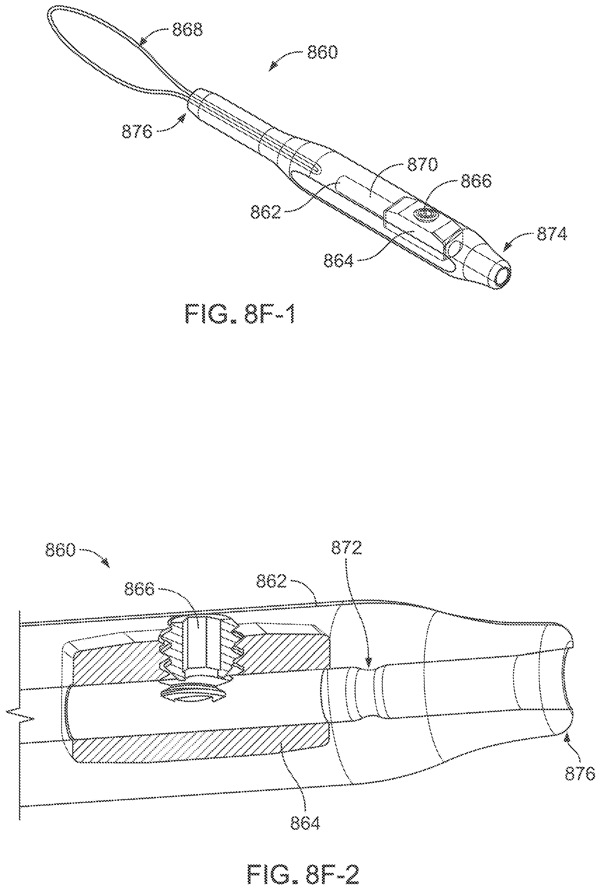

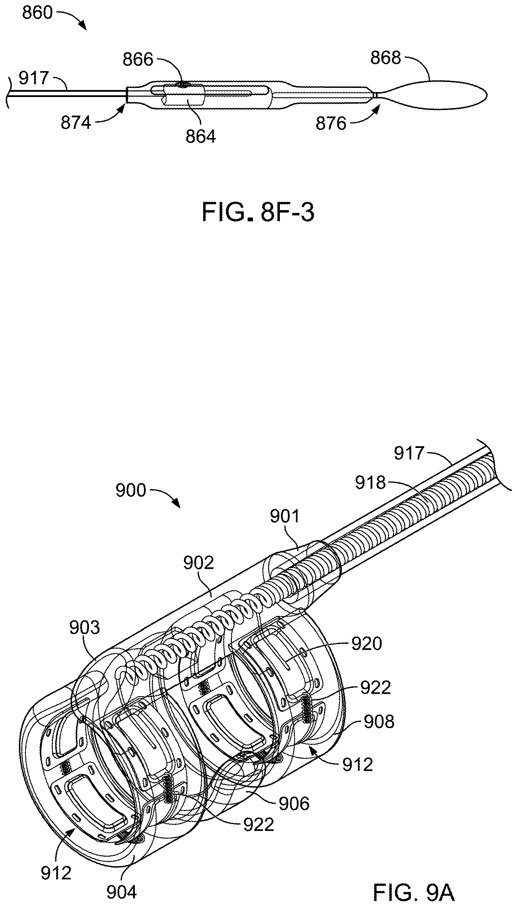

FIG. 8F-1 is a perspective view of an embodiment of a lead cap device.
FIG. 8F-2 is a partial cross-sectional view of the lead cap device of FIG. 8F-1.
FIG. 8F-3 is another perspective view of the lead cap device of FIG. 8F-1.
FIG. 9A is a perspective view of an embodiment of an electrode device.

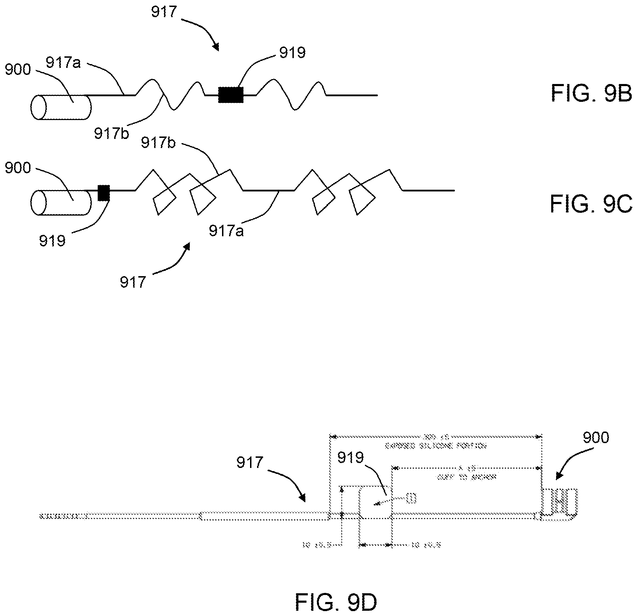

FIG. 9B is a line diagram of an electrode device and lead body according to an embodiment.
FIG. 9C is a line diagram of an electrode device and lead body according to another embodiment.
FIG. 9D is a side view of an electrode device and lead body according to an embodiment.

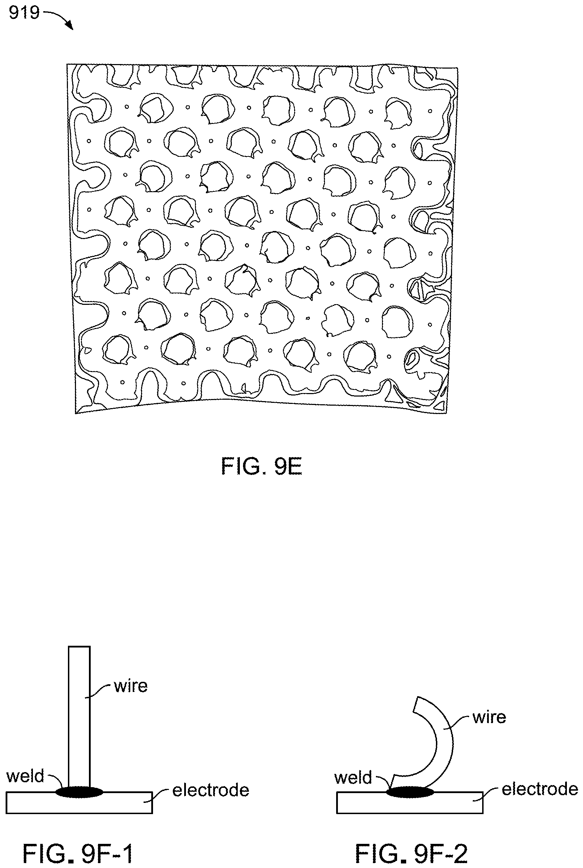

FIG. 9E is a photographic view of a coated mesh structure of an anchoring tab according to an embodiment.
FIG. 9F-1 is a side view line diagram of a weld interface between a wire and an electrode according to an embodiment.
FIG. 9F-2 is a side view line diagram of a weld interface between a wire and an electrode according to another embodiment.

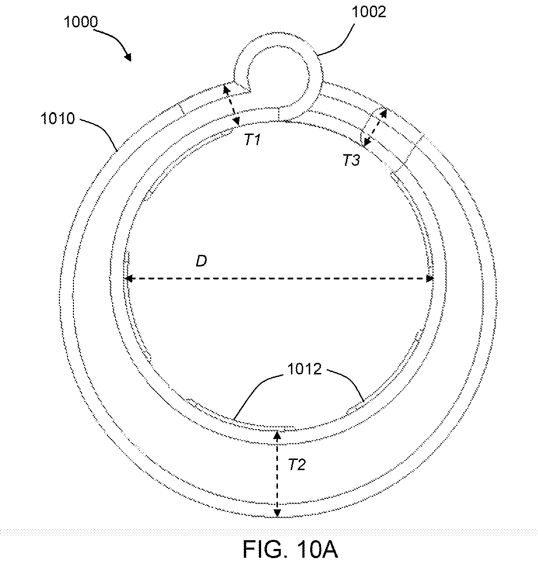

FIG. 10A is an end view of the electrode device of FIG. 9A according to an embodiment.

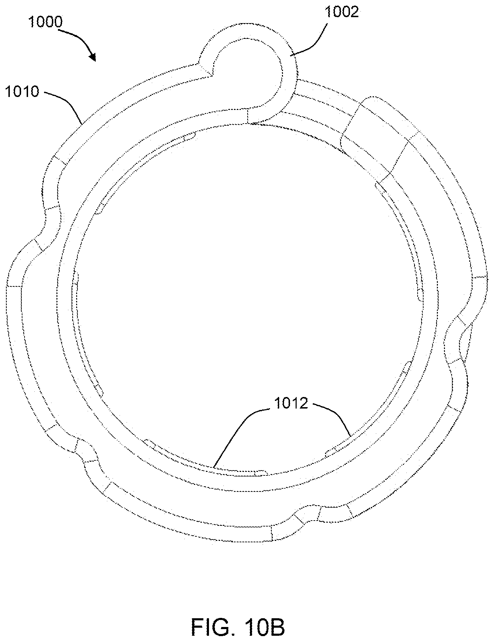

FIG. 10B is an end view of the electrode device of FIG. 9A according to another embodiment.

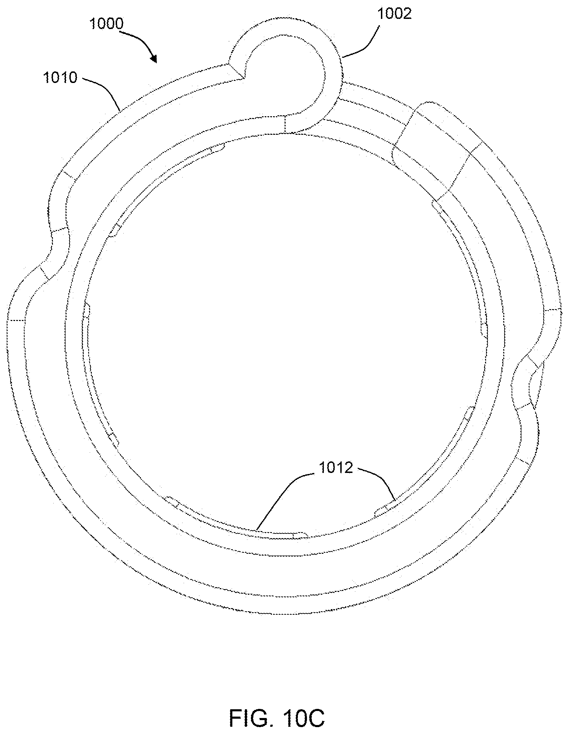

FIG. 10C is an end view of the electrode device of FIG. 9A according to yet another embodiment.

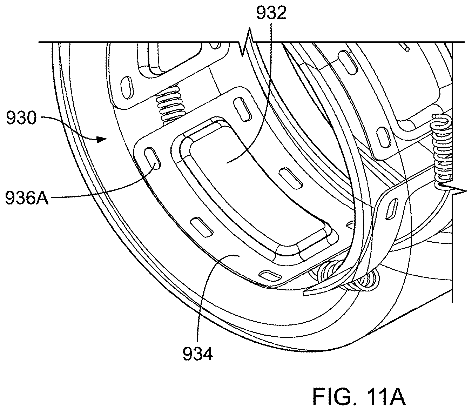

FIG. 11A is a partial perspective view of an electrode of the electrode device of FIG. 9A.

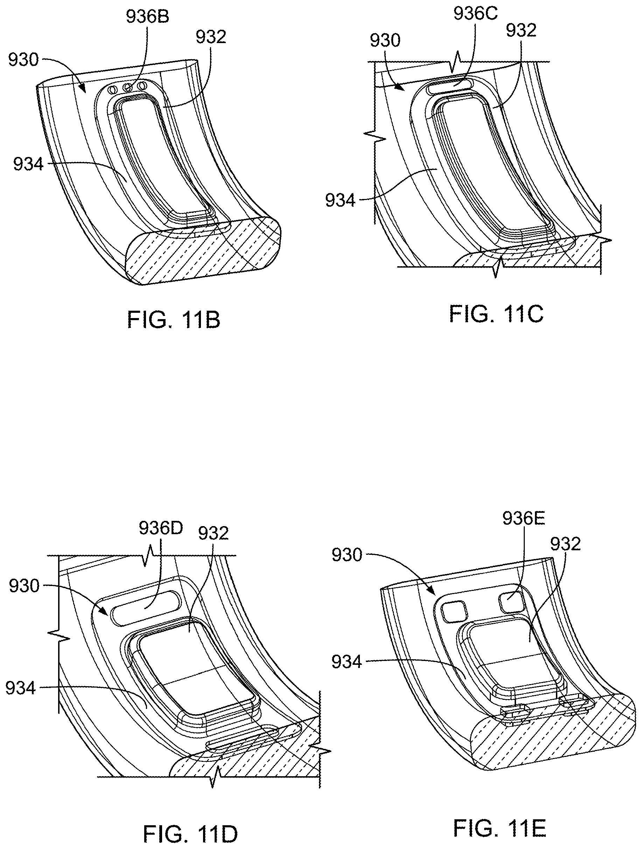

FIG. 11B is a partial perspective views of an electrode and an electrode device cuff portion according to an embodiment.
FIG. 11C is a partial perspective views of an electrode and an electrode device cuff portion according to an embodiment.
FIG. 11D is a partial perspective views of an electrode and an electrode device cuff portion according to an embodiment.
FIG. 11E is a partial perspective views of an electrode and an electrode device cuff portion according to an embodiment.

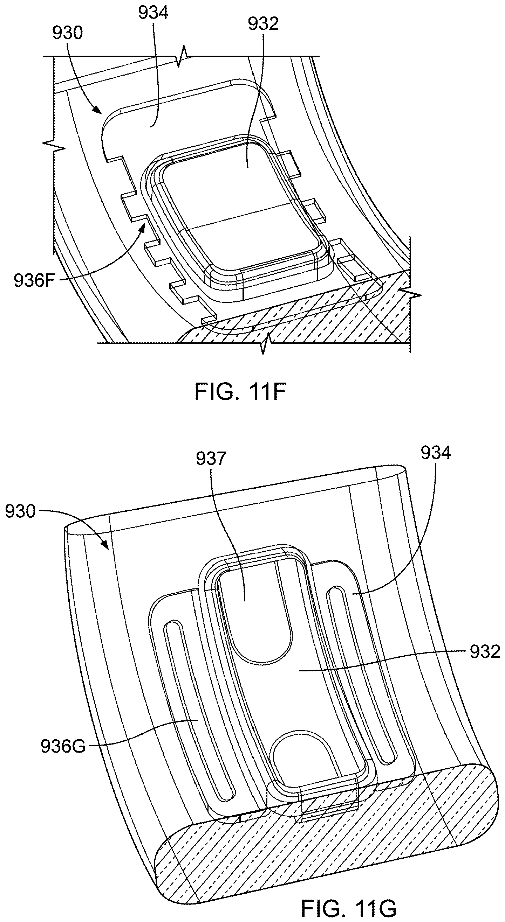

FIG. 11F is a partial perspective views of an electrode and an electrode device cuff portion according to an embodiment.
FIG. 11G is a partial perspective views of an electrode and an electrode device cuff portion according to an embodiment.

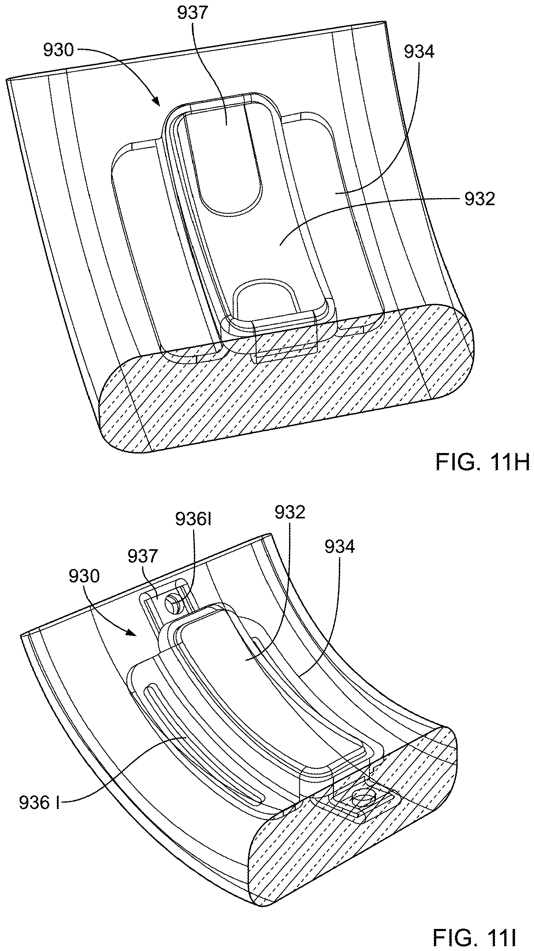

FIG. 11H is a partial perspective views of an electrode and an electrode device cuff portion according to an embodiment.
FIG. 11I is a partial perspective views of an electrode and an electrode device cuff portion according to an embodiment.

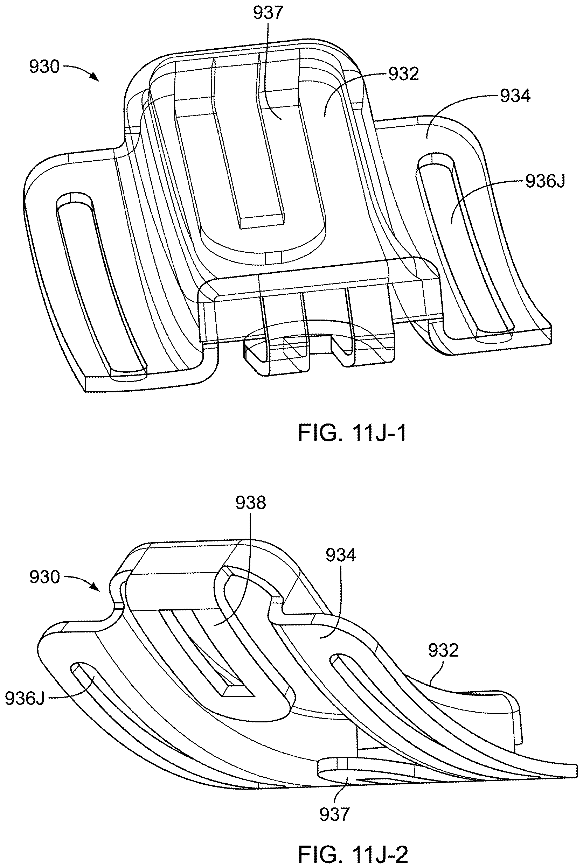

FIG. 11J-1 is a partial perspective views of an electrode and an electrode device cuff portion according to an embodiment.
FIG. 11J-2 is a partial perspective views of an electrode and an electrode device cuff portion according to an embodiment.

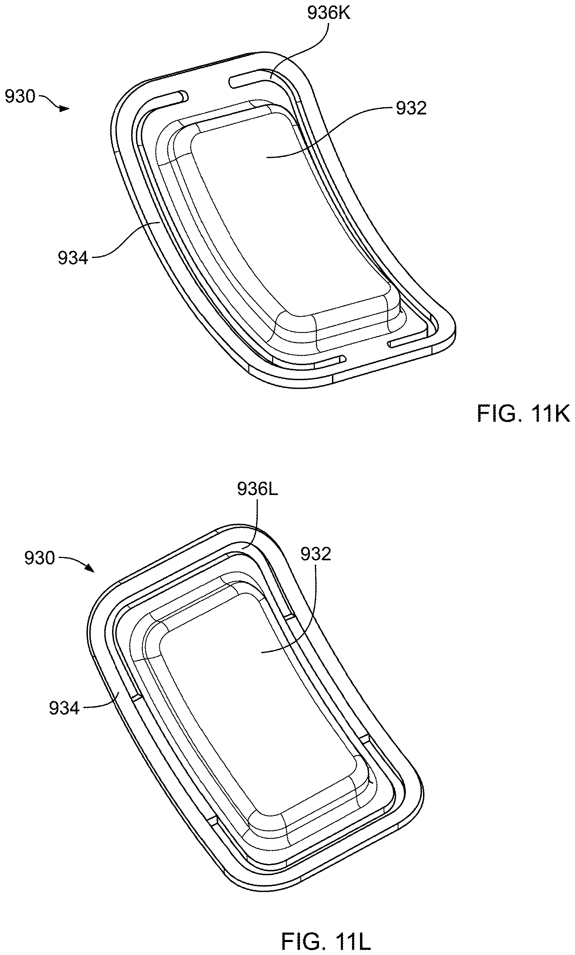

FIG. 11K is a partial perspective views of an electrode and an electrode device cuff portion according to an embodiment.
FIG. 11L is a partial perspective views of an electrode and an electrode device cuff portion according to an embodiment.

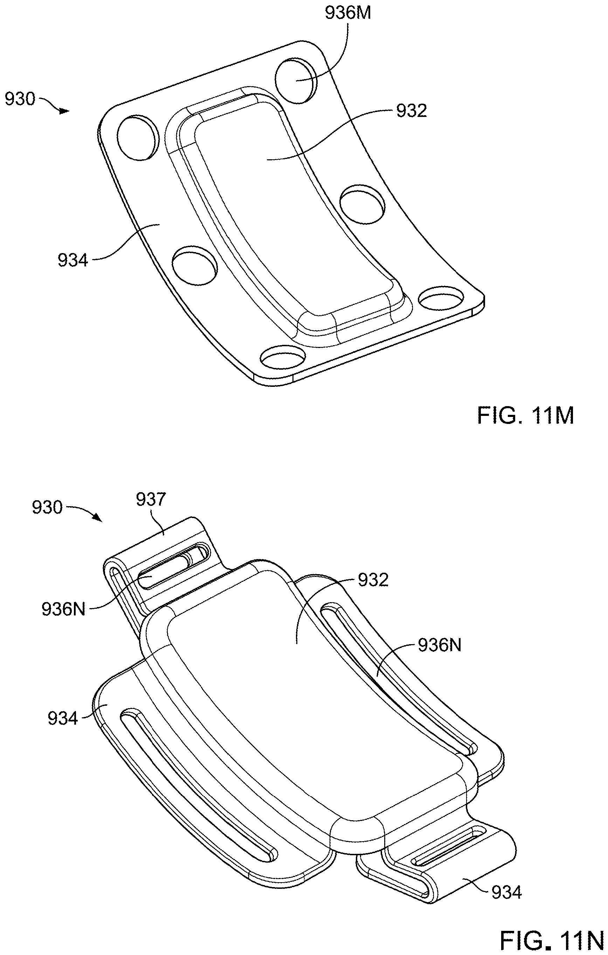

FIG. 11M is a partial perspective views of an electrode and an electrode device cuff portion according to an embodiment.
FIG. 11N is a partial perspective views of an electrode and an electrode device cuff portion according to an embodiment.

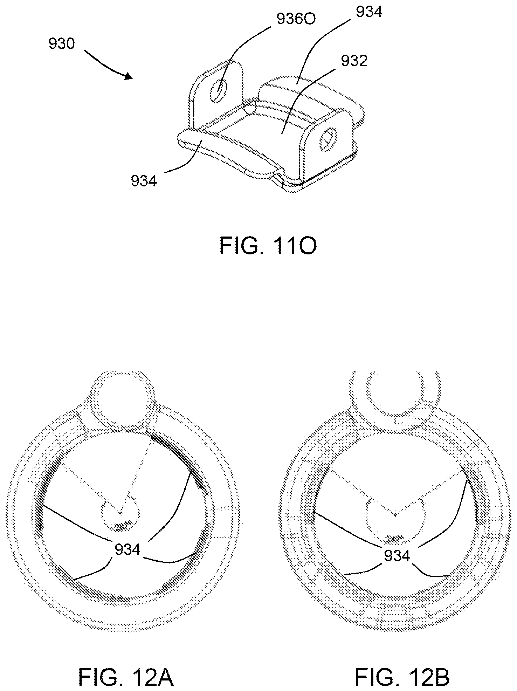

FIG. 11O is a partial perspective views of an electrode and an electrode device cuff portion according to an embodiment.
FIG. 12A is an end view of an electrode device according to an embodiment.
FIG. 12B is an end view of an electrode device according to another embodiment.

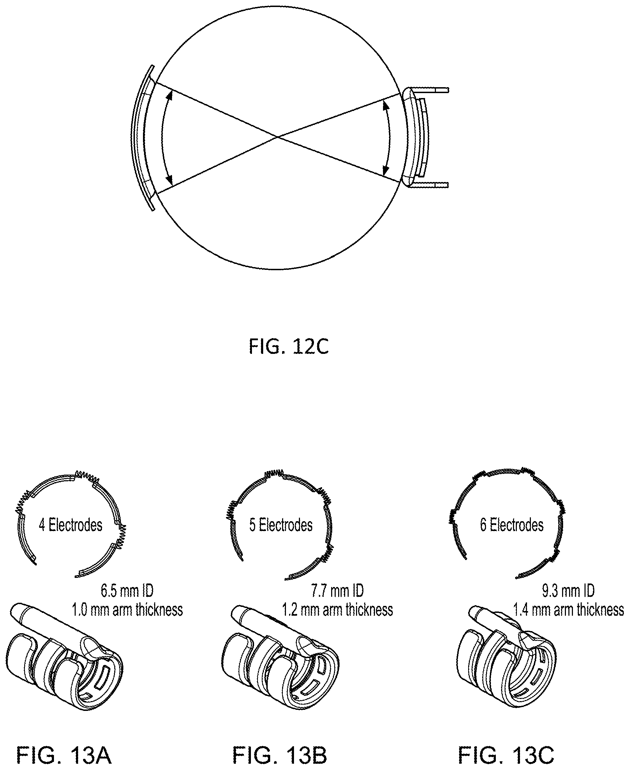

FIG. 12C is an end view showing an electrode of the embodiment of FIG. 12A and an electrode of the embodiment of FIG. 12B.
FIG. 13A is a perspective view of a smaller cuff and electrode arrangement according to an embodiment.
FIG. 13B is a perspective view of a midsize cuff and electrode arrangement according to an embodiment.
FIG. 13C is a perspective view of a larger cuff and electrode arrangement according to an embodiment.

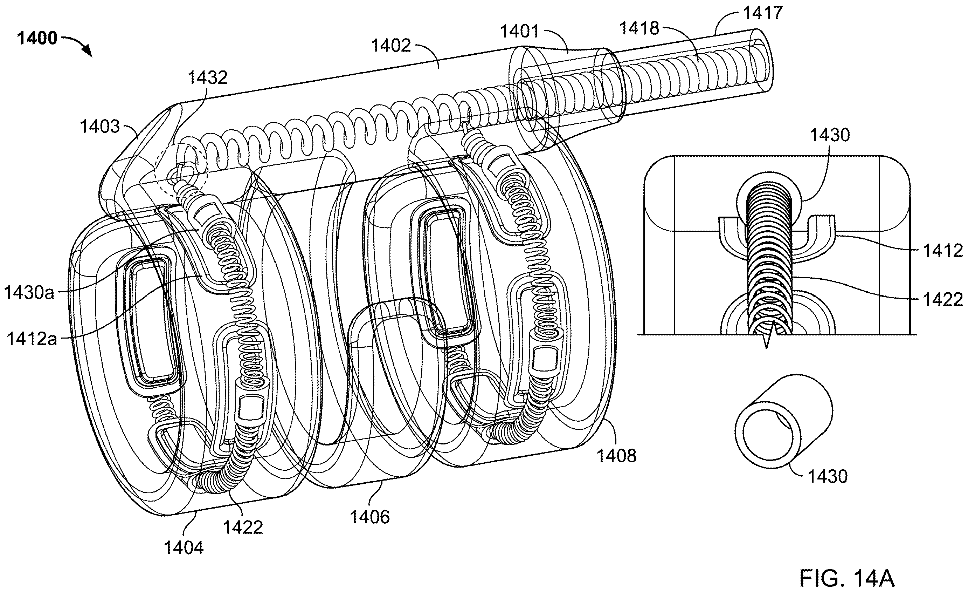

FIGS. 14A-14F depict embodiments in which different interconnection arrangements are used to provide power to the electrodes.

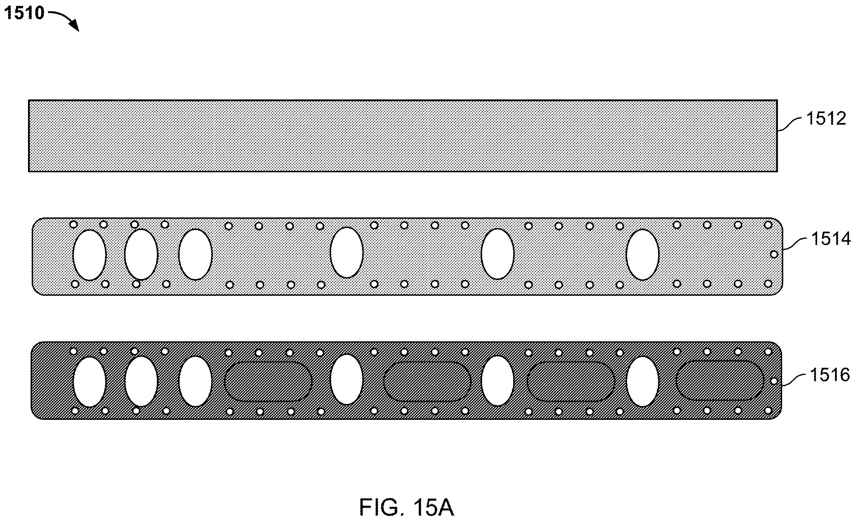

FIGS. 15A - 15I depict example embodiments in which a unitary electrode array with increased flexibility between electrodes within the array is provided.

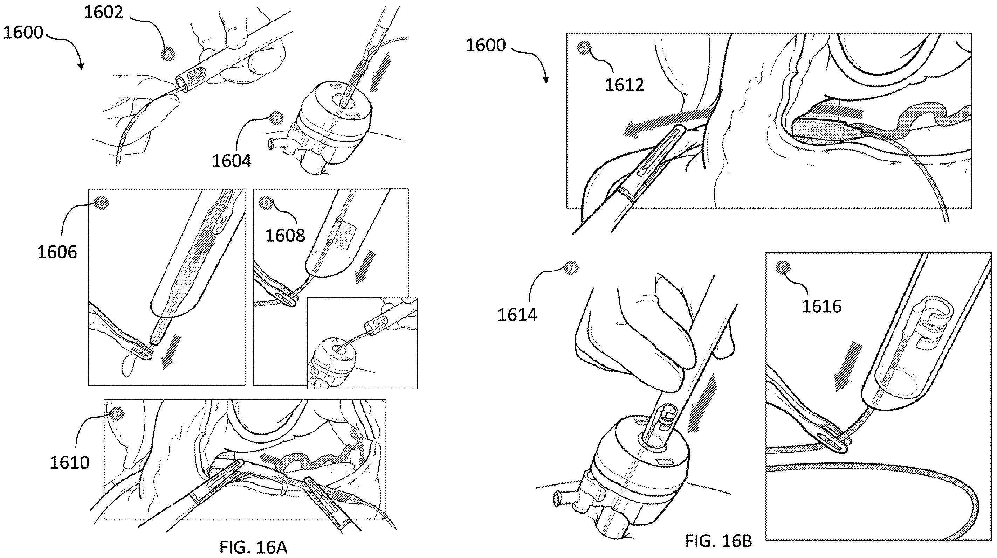

FIGS. 16A and 16B depict a method for installing devices described herein.

### DETAILED DESCRIPTION OF THE DRAWINGS

The present disclosure is related to embodiments of extravascular and intravascular neural interface devices containing electrodes for neurostimulation/neuromodulation of a target nerve or vessel. The devices may be housed in flexible substrates, each substrate having a central portion through which conductors for the electrodes are routed and housed. Extending from the central portion are a plurality of curvilinear flaps or arms that support the electrodes and position the electrodes to either be inward facing, i.e., extravascular designs, or outward facing, i.e., intravascular designs. An extravascular neural interface device is configured to be positioned outside of the target vessel, while an intravascular neural interface device is configured to be positioned at least partially within the target vessel. The substrate flaps or arms may include one or more electrodes and be configured to place one or more of the electrodes at specific positions relative to the target vessel.

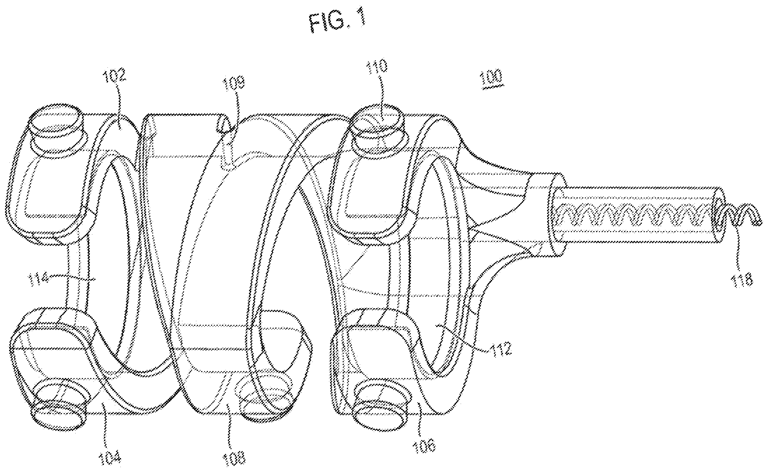

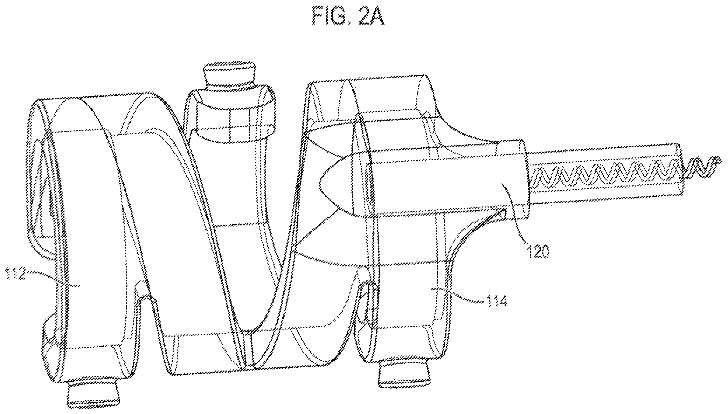

An embodiment of a bi-polar, extravascular neural interface in accordance with the present disclosure is illustrated in FIGS. 1 and 2A. The neural interface 100 may comprise a hybrid cuff, including a partially-helically formed supporting substrate 102, manufactured of silicone or a similar flexible substance, such as styrene isoprene butadiene (SIBS), polyamide, parylene, liquid-e1ystal polymer (LCP), polytetrai1uoroethylene (PTFE), polyethylene (PE), polypropylene (PP), fluorinated ethylene propylene (FEP), ethylene-tetrafluoroethylene (ETFE), polyurethane, or another biocompatible polymer. Biocompatible silicone and some other grades of silicone can be very flexible and soft, thereby minimizing mechanical mismatches between a cuff and a target vessel and minimizing constriction on the target vessel. Polymer materials may also be used, but those materials may be stiffer and harder than silicones and may require thinner material to be used, which may be both an advantage and a drawback.

The substrate 102 may include two C-ring portions 104 and 106, each connected by a spinal portion forming a helix of one turn (when combined with a center portion 109 and one of the portions 104 or 106) in the opposite direction from a common center section 108 of a central portion 109, and ending in an C-ring configuration where the C-ring is substantially orthogonal to the target vessel once positioned. Arranged within each C-ring end portion 104 and 106 may be multiple platinum or platinum alloy electrodes (or electrode arrays), such as electrode arrays 112 and 114, versus multiple helical structures as in conventional systems. The electrodes arrays 112 and 114 may be of a conventional type and wired to a controller through conventional conductors 118, such as 35N LT^{®} DFT (Drawn Filled Tubing) with a 28% Ag core, in a stranded cable configuration (i.e., 7x7 configuration- not shown), or in a multi-filar coil configuration. The conductors are housed in a spine or spinal portion 120 that is affixed to end portion 106 and part of central portion 109.

The configuration of the neural interface 100 may make it possible to significantly shorten the length of the neural interface 100, thereby reducing the portion of target vessel or nerve that needs to be mobilized during placement. In addition, the opposing helical directions of the portions 104 and 106, which each have a low helix angle relative to the spinal portion 120 may allow the neural interface 100 to be deployed and wound around the target vessel in one pass instead of at least two, as with conventional helical structures. A low helix angle or low pitch may allow the length of the neural interface 100 (or its distal end) to be shorter, which may result in less dissection of tissue during positioning.

The substrate 102 may include a number of attributions 110 placed at different points on or in the substrate 102. The attribution may be configured to enable a deployment tool (not shown) to grip, manipulate and deploy the neural interface 100. Thus, each attribution may be referred to as a deployment feature. The attributions may be protrusions. One or more protrusions may include one or more openings or eyelets for receiving a stylet (made of tungsten or similar material), for instance, in order to enable portions of the substrate to be straightened or to deploy the neural interface. The attributions 110 may also be openings, eyelets or some other form of lumen that may be equally manipulated by a deployment tool.

In an embodiment, the attributions 110 may be placed sufficiently near the open ends of the C-rings of portions 104 and 106 and near the end of center section 108 to enable the deployment tool to grip the attributions and simultaneously open the portions 104 and 108 and the center section 108 so that the neural interface may be positioned around the target vessel (not shown). As referred to herein, "open ends" refer to the ends positioned around the circumference of the C-rings, which are not attached to another feature (e.g., another C-ring or a spinal portion). In other words, each one of the "open ends" forms a side of a gap for the target vessel. Similarly "closed ends" refer to ends positioned around the circumference of the C-rings which are attached to another feature. In other words, each one of the "closed ends" does not form a gap for the target vessel. Once the neural interface 100 has been positioned around the target vessel, the deployment tool would carefully release the attributions so that the portions 104 and 108 and the center section 108 may softly self-size to the target vessel. By "self-size" it is meant that the neural interface 100 conforms to the shape of the target vessel of its own accord.

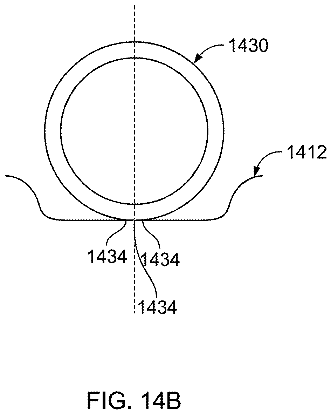

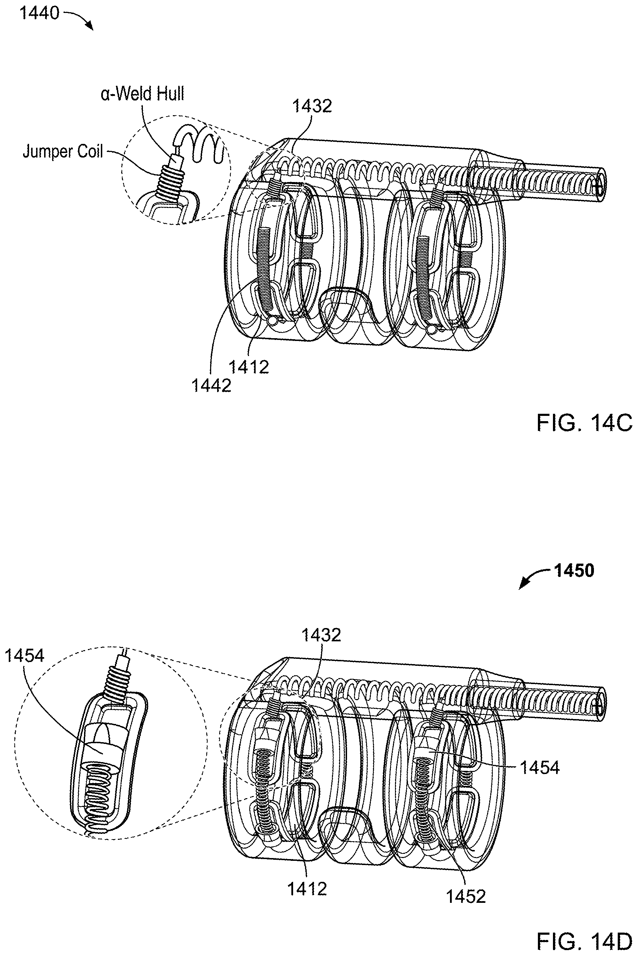

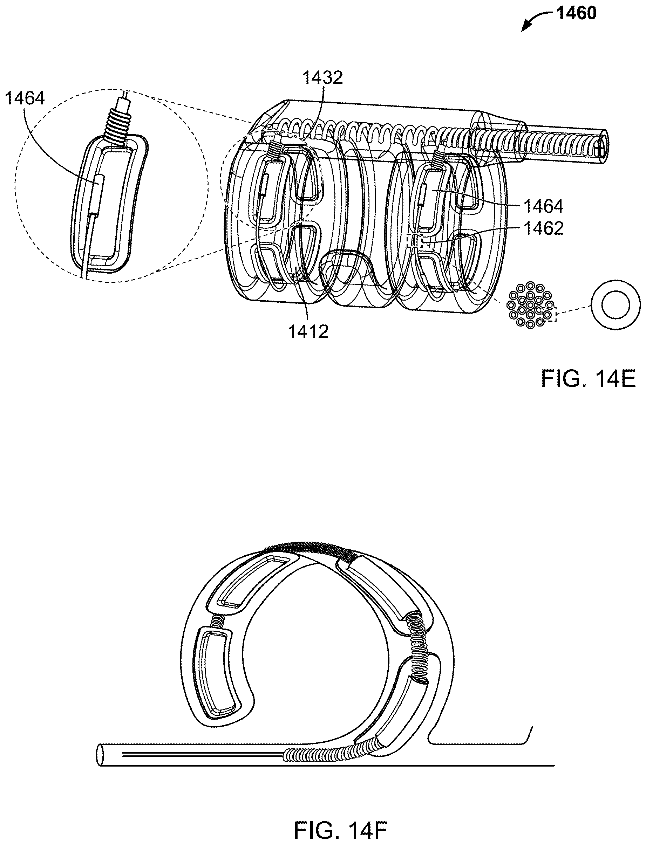

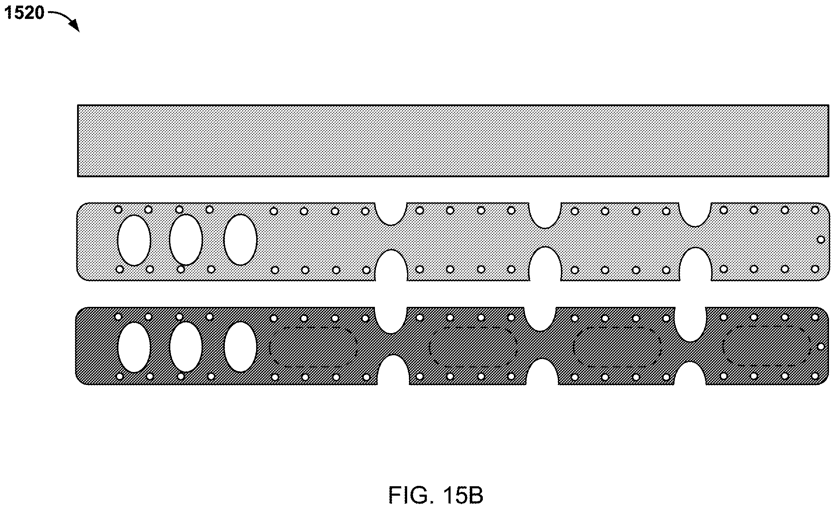

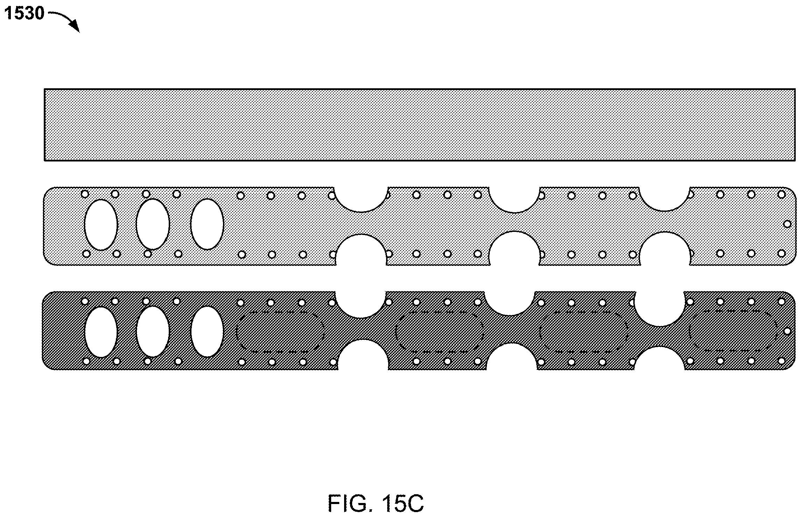

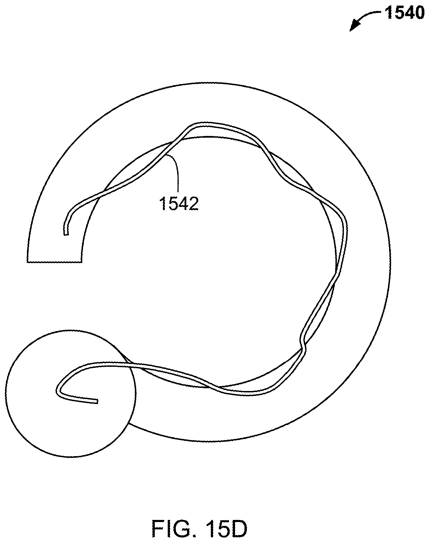

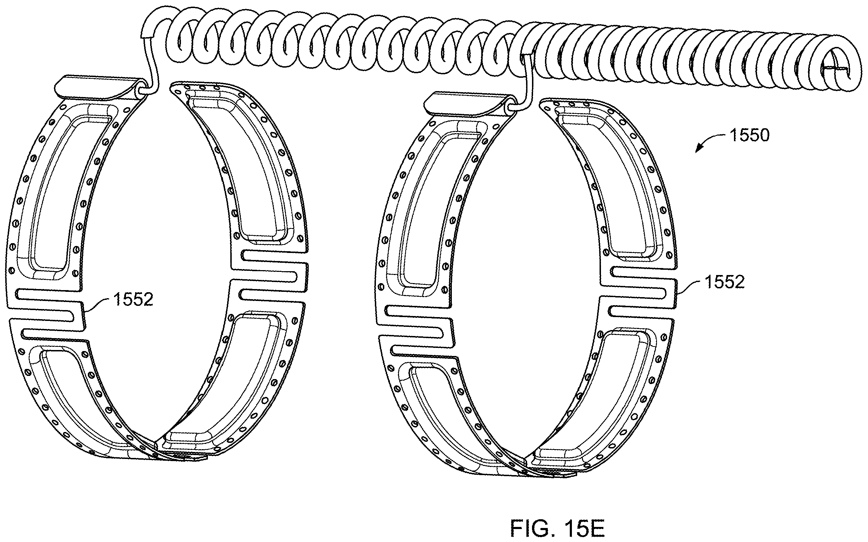

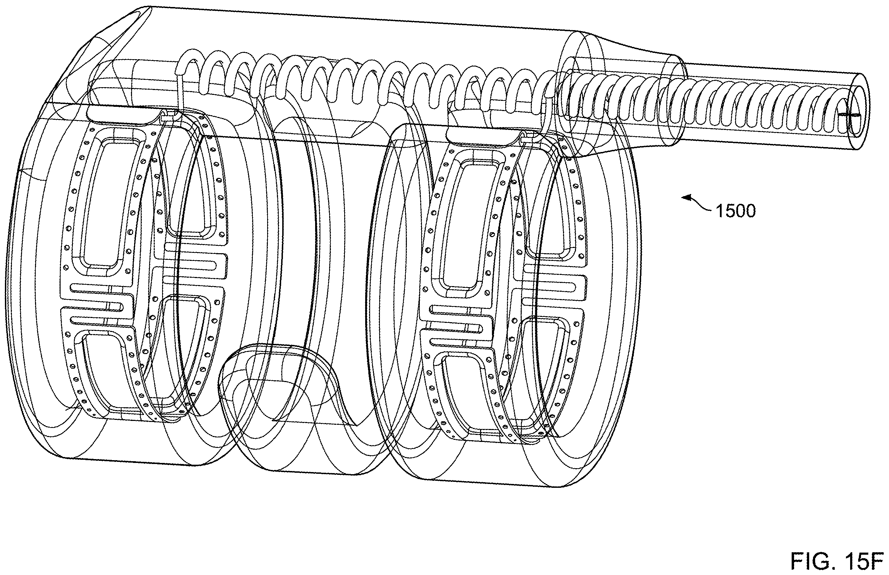

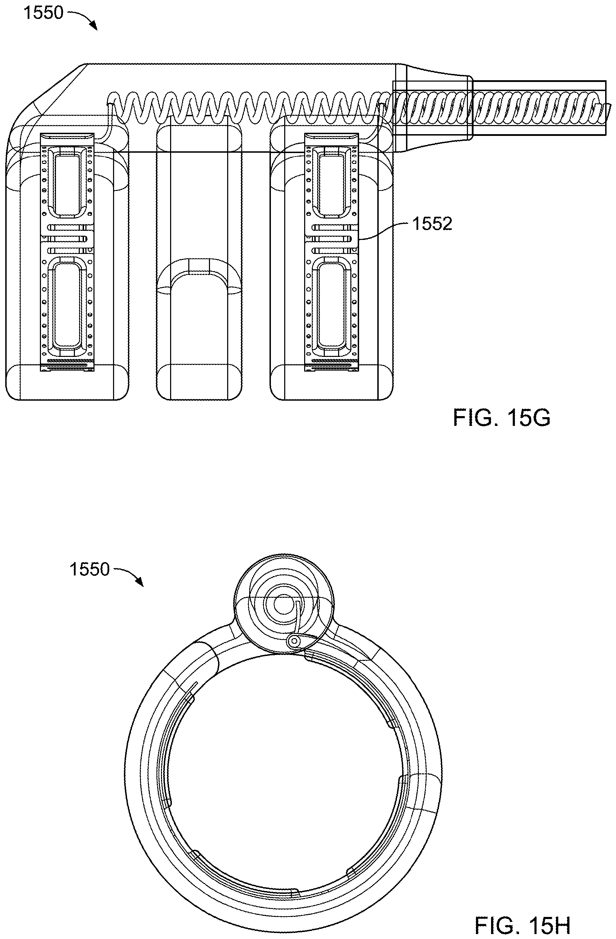

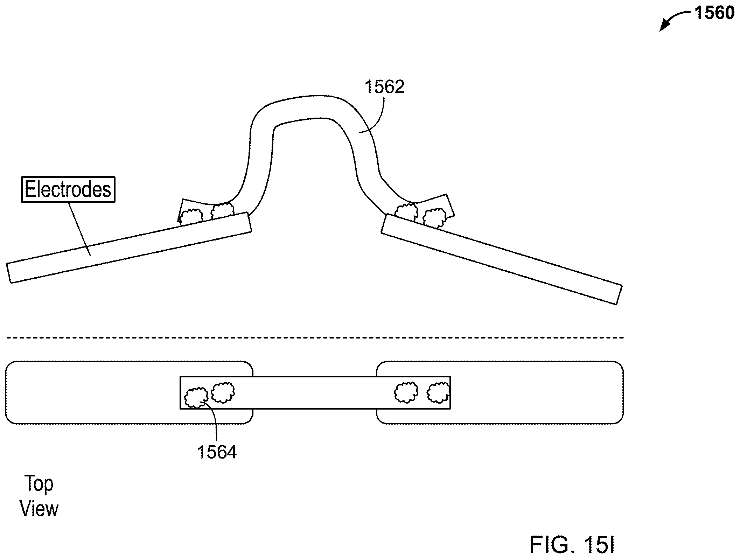

The C-ring can exhibit a radial pressure on the target vessel, nerve, or other structure. The amount of pressure applied can depend on a number of factors. The material that makes up the C-ring portions is, for example, an insulative material and an electrically conductive portion (such as an electrode, a coil, foil, or weldments as described below) that passes therethrough or is exposed at least in part (such as an electrode). The C-ring portion applies a radial pressure that is a function of a number of features, such as the rigidity of an insulating material that makes up a body of the C-ring portion (for example 70-80 Shores). The thickness of an insulating material that makes up the body of the C-ring portion also affects the radial pressure, as does the rigidity or hardness of electrode or electrodes passing through the C-ring. As described in more detail below with respect to FIGS. 14 and 15, a size and shape of the at least one electrode can affect the flexibility of the electrode, and therefore affect the radial pressure applied. A quantity of electrodes on any given C-ring may also affect the radial pressure applied. A target facing surface of the C-ring can be formed of an active portion (also referred to as an exposed electrode portion) and an inactive portion (also referred to as the insulating material portion), and a ratio of the active portion and the inactive portion can affect the radial pressure. For example, a ratio of the active portion to the inactive portion (i.e. active portion : inactive portion) may be between 1:0.5 to 1:10. In preferred embodiments, the ratio may be between 1:1.5 - 1:5, or 1:3. A gap size between any given pair of electrodes in a C-ring also affects the pressure applied, as described in more detail below. The properties of the interconnect between different electrodes can make an impact on radial pressure.

Thickness of the C-ring material is, for example, between about 0.5mm - 2mm, such as about 1 mm, about 1.2mm, or about 1.4mm, in some embodiments. The thickness is defined along the radial direction from the central axis shown in FIG. 1, for example. Referring again to FIG. 1, the diameter of the cuff (i.e. length of the C-ring material about the central axis) can be between 1mm and 25mm, preferably between about 3 mm and about 10mm, such as 4, 5, 6, 7, 8, or 9mm or any number in between, further preferably between about 4mm and 8mm in embodiments. However, this can also be chosen based on the target size.

As shown in FIG. 9A (as well as the additional embodiments described below), each device has an inner diameter and an outer diameter. The term "inner diameter," used herein to describe such devices, refers to the distance from a central axis to a radially inward-facing portion of the device, on which the electrodes are arranged. The "outer diameter," in contrast, is the distance to the radially outer portion opposite those electrodes, which defines the furthest radial extent of the device. The outer diameter is the furthest radial extent of the C-ring portions, and is not defined by the radially outermost extent of other protuberances such as a lead body or other feature attached to the radially outer face of the C-ring portions. The C-ring portions of neural interfaces described herein have an inner diameter and a cross-sectional thickness, with a ratio of the inner diameter to the cross-sectional thickness being in a range of 4:1 to 9:1, or in a range of 5:1 to 7: 1, or 5:1 to 6:1 in some embodiments. In other embodiments, such as when thin films are used, the ratio can be as high as 40:1, though the ratio may be closer to 10:1 for increased structural integrity. On the other hand, where higher radial force is desired the ratio could be as low as 3:1. It will be understood by the skilled person that the ratio may also be chosen in relation to the stiffness of the material used. For example, with a stiffer material having a higher Shore number, a higher ratio may be used to achieve a similar radial force as those with a less stiff material but a lower ratio.

Depending on the above discussed physical aspects, and depending upon the resting radius of the C-ring relative to the nerve or vessel that it is applied to (i.e., how much the C-ring is expanded), the radial pressure exerted by the C-ring can be determined as an average contact pressure. The term "resting radius" used herein refers to the inner radius of the device when no external force is applied to it. A device may have a different resting radius when positioned on a target, as the target can maintain an enlarged radius compared to the resting radius in isolation, biasing the device to a relatively more open position. This average contact pressure can be between about 0 mmHg and about 30 mmHg. In some embodiments, the average contact pressure can be between about 5 mmHg and about 25 mmHg, or between about 5 mmHg and about 20 mmHg, or between about 10 mmHg and about 20 mmHg, or between about 10 mmHg and about 15 mmHg. In preferred embodiments, the C-ring will exhibit at least some radial pressure (e.g., at least 1 mmHg) but will not exert so much pressure as to damage the underlying anatomical structure.

The change in radial size can correspond directly to the amount of radially applied force. Radial pressure, as referred to herein, corresponds to an applied pressure when the C-rings or the cuff are opened by about 0%-40% (i.e., 0-40% increase in the diameter of the cuff when deployed on a target compared to the original cuff diameter size). In other words, the average radial pressure exerted by the C-ring is between about 0mmHg and 30mmHg when the cuff diameter expansion is anywhere between 0%-40%. In some embodiments, the average radial pressure exerted by the C-ring is between about 0mmHg and 30mmHg when the cuff diameter expansion is anywhere between 0% and 35%. In some embodiments, the average radial pressure exerted by the C-ring is between about 0mmHg and 30mmHg when the cuff diameter expansion is anywhere between 0% and 30%. In some embodiments, the average radial pressure exerted by the C-ring is between about 0mmHg and 30mmHg when the cuff diameter expansion is anywhere between 0% and 25%. In some embodiments, the average radial pressure exerted by the C-ring is between about 0mmHg and 30mmHg when the cuff diameter expansion is anywhere between 0% and 20%. Preferred expansion may be between 10%-30% in some embodiments for desirable electrical contact and average radial pressure. Expansion beyond 40% typically occurs only during deployment or removal of the C-ring from the target.

Detachment force or retention force refer to the force required to remove the device from the target after it has been placed at least partially around the target anatomical structure. Detachment force can be between 0.05N-0.5N in some embodiments. In a preferred embodiment, detachment force can be between 0.1-0.2N. In a preferred embodiment, a detachment force of about 0.15N when pulled in perpendicular direction from the target axis is sufficient to remove the device from the target.

Whilst embodiments comprising three open ended arms are discussed as an example and depicted, various aspects of the present disclosure may be applied to neural interface with different shapes or arrangements. For example, the neural interface may comprise only one open ended arm, two open ended arms, or more than three open ended arms. Further, the neural interface may comprise arms with the same coupling and opening orientations, alternating coupling and opening orientations, or other patterns of arm coupling and arm orientations; arms of different relative sizes; arms of different or varying helical angles; and other variations, including as discussed herein with respect to other embodiments.

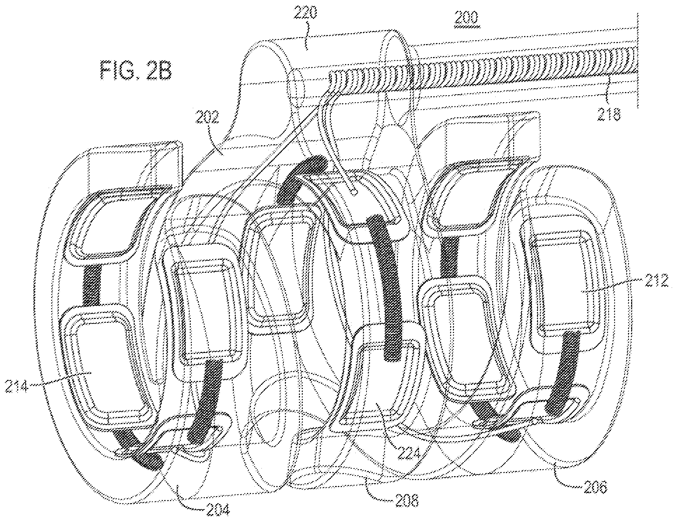

Another embodiment of a neural interface 200, similar in structure to the embodiment illustrated in FIGS. 1 and 2A, i.e., with multiple C-rings, a common center section, and forming two helical turns over a short length, is illustrated in FIG. 2B. Neural interface 200 may be multipolar instead of bipolar as may be the case with neural interface 100. In neural interface 200, the substrate 202 may include three C-ring portions 204, 206 and 208, with each C-ring end portion 204 and 206 connected by a one turn helix in the opposite direction from a common center section of an C-ring central portion 208, and ending in C-ring configurations that may be orthogonal to the target vessel once positioned on the target vessel. C-ring central portion 208 may also be orthogonal to the target vessel. Positioned within each C-ring portion 204, 206 and 208 may be multiple platinum or platinum alloy electrodes (or electrode arrays), such as electrode arrays 212, 214 and 224, fashioned (arranged) in such a way that electrodes in one C- ring covers the gap (along a length between electrodes) in the adjacent C-ring. Each electrode array is connected to a different conductor of the multi-conductor 218 housed in spinal portion 220, which is affixed to just central portion 208. The substrate 202 of neural interface 200 may not include attributions. Connecting individual electrodes or different arrays of electrodes to different conductors may enable selective stimulation of the target vessel by individually controlling each connected device or individual electrode or individual groups of electrodes.

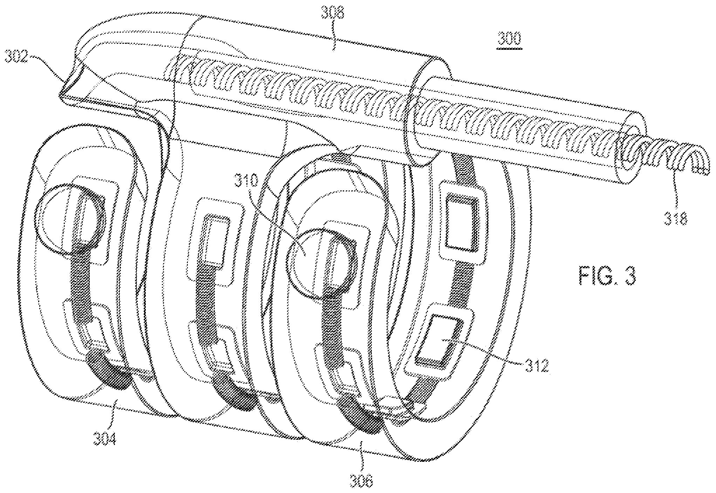

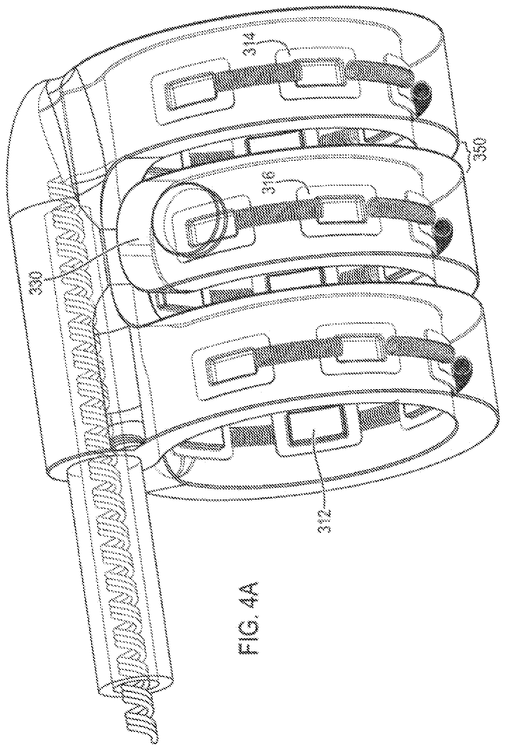

FIGS. 3 and 4A illustrate an embodiment of a tripolar neural interface 300 in accordance with the present disclosure. The neural interface 300 may be similar to neural interface 100 in that it may be formed of a flexible substrate 302 of similar material and may have two end portions 304 and 306 forming C-ring configurations that may be affixed to a spinal portion 308.

However, unlike neural interface 100, the two end portions 304 and 306 may also not be connected to the center section. Instead, a center portion 330 forming a third C-ring may be utilized. The end portions 304 and 306 and the center portion 330 may have a very low helix angle, i.e., pitch, relative to the spinal portion 308, which enables the neural interface to be helical, but still have a significantly shorter length. The helix angle may be between approximately 15 and 30 degrees, but may also be less than 15 degrees.

As with neural interface 100, each of the C-rings of the neural interface 300 may include one or more electrodes or an array of electrodes, such as 312, 314 and 316, each connected to a conductor 318 through the spinal portion 308. A one-electrode design may make it possible to maximize electrode coverage while minimizing the conductor interconnection process, such as through laser welding, resistance welding, etc. However, to minimize the rigidity of the electrode, i.e., making it sufficiently flexible, the electrode may have to be very thin (typically between 25 µm and 50 µm), which may make the interconnection of the conductors to the electrodes more challenging. Also, to keep the electrode as flexible as possible, surface features may not be possible to add to the electrode as it would decrease the electrode flexibility. For this reason, a one electrode may feature recessed electrodes with silicone rims or silicone webbing that may serve to hold the electrode in place. However, recessing the electrode may potentially decrease the efficacy of the stimulation. On the other hand, "segmented" electrode designs may provide better mechanical compliance, create the possibility of surface features, i.e., protruding electrodes, and make it possible to control each electrode individually (i.e., current steering). The trade-offs include limited electrode coverage, increased interconnection processes, decreased retention force. Segmented electrodes provide increased flexibility to the neural interface, thereby making it possible open a C-ring with a deployment tool wider and for a longer period of time, without creating excessive stress on the electrodes, than might be possible with a single electrode.

As shown in FIGS. 2A and 4A, the individual electrodes of the electrode arrays 112 and 114 of neural interface 100 and electrode arrays 312, 314 and 316 of neural interface 300 may be evenly spaced within the substrates 102 and 302, respectively. By evenly spacing the electrodes within the substrates, the inter-electrode distance is more constant, which may provide a more uniform current density distribution and enhancement of the effectiveness of the neural interfaces. In some embodiments, the position of the electrodes in the arrays may be staggered in order to achieve better electrical coverage. Certain characteristics of the neural interfaces 100 and/or 300, such as, with respect to neural interface 300, the spacing 350 between the electrode arrays 312, 314 and 316, the size and shape of the electrodes, the size and shape and number of electrodes in electrode arrays, the inter-electrode distance within electrode arrays, and the angle of the helix angle, may each be chosen for the particular application of the neural interface. For example, utilization of the neural interface for treatment of the splenic artery may require different characteristics than utilization of the neural interface for treatment of a different vessel. For instance, when utilized for splenic artery treatment an electrode width of approximately 1-4 mm, with preferred width ranges of between approximately 1-2 mm and approximately 2-3 mm, may be appropriate. When utilized for treatment of different vessels, different electrode widths may be desirable.

The neural interface 300 may also include at least one attribution 310 that may be positioned on the outer surface of the substrate 302 near the open ends of each C-ring of the portions 304, 306 and 330. As noted above, the attributions may include one or more openings or eyelets for receiving a stylet (made of tungsten or similar material), for instance, in order to enable portions of the substrate to be straightened and/or to deploy the neural interface. The attributions 310 may be configured to enable a deployment tool (not shown) to grip, manipulate and deploy the neural interface 300. In an embodiment, the attributions 310 may be placed sufficiently near the open ends of the C-rings of portions 304, 306 and 330 to enable the deployment tool to grip the attributions 310 and simultaneously open the portions 304, 308 and 330 so that the neural interface 300 may be positioned around the target vessel (not shown). Once the neural interface 300 has been positioned around the target vessel, the deployment tool would carefully release the attributions so that the portions 304, 308 and 330 may softly self-size to the target vessel. The configuration of the neural interfaces 100 and 300 may enable the neural interfaces to be positioned in a single pass around the nerve/vessel with minimal manipulation of the nerve/vessel and a reduction in tissue dissection around the area of the nerve/vessel where the interface is positioned.

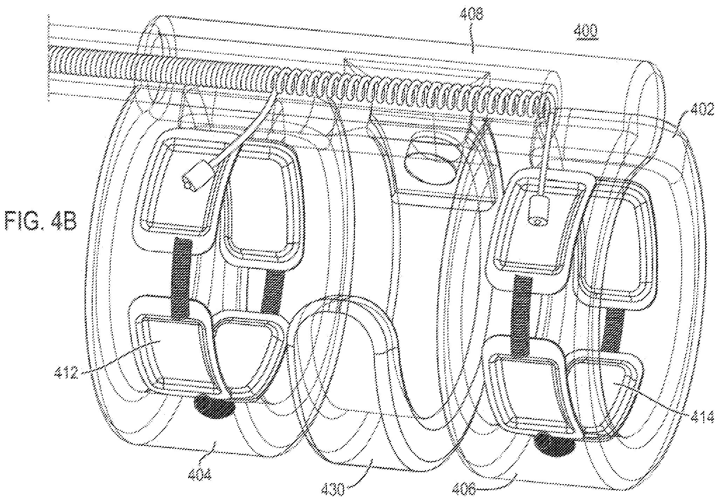

The neural interface 400 in FIG. 4B is similar to the neural interface 300. Neural interface 400 is formed of a flexible substrate 402 of similar material and may have two end portions 404 and 406 forming C-ring configurations containing electrode arrays 412 and 414. A center portion 430 may be affixed to a spinal portion 408 along with end portions 404 and 406.

The center portion 430 may not include any electrodes, serving just to retain the neural interface once positioned, but embodiments may include electrodes.

The neural interfaces 100, 200, 300 and 400 may be self-sizing; meaning that they may be formed of flexible materials that allow them to be manipulated for deployment, but when released return to a predetermined shape, much like a nitinol cage can be reduced down to fit in a catheter and return to its pre-reduced shape once released from the catheter. This may enable the neural interfaces to be used to accommodate anatomical variability of the intervention site, yet still provide good electrical contact between the electrode arrays and the surface of the nerve/vessel, thereby improving the efficient of the interface. The flexible material of the interface may remain compliant even when it has self-sized to a nerve or vessel. This may help to prevent the neural interface from compressing a nerve or vessel and causing reduced blood flow and otherwise constricting nerve fiber. This may also better accommodate radial expansion of the nerve/vessel as a result of post-positioning edema or swelling and may accommodate the pulsatile behavior of intervention sites such as arteries.

The naturally open structure of the helix of the neural interfaces 100, 200, 300 and 400 may reduce coverage of the nerve/vessel periphery to promote more normal fluid and nutrient exchange with the intervention site and surrounding tissue. This may also help to minimize growth of connective tissue between the electrode nerve/vessel interfaces. The open structure of each neural interface is configured such that no end portion or center portion forms a closed circumscribed circular arc around the target vessel at any point along a length of the target vessel. In other words, no closed circle covering 360 degrees of an orthogonal portion of the target vessel's length is formed by the structure. This open unrestricted trench may serve to ensure that the target vessel may pulsate without constriction and that an initially swollen target vessel can return to a normal state over time without constriction of the target vessel when it is swollen and without losing electrode to target vessel contact when the target vessel is in its normal state.

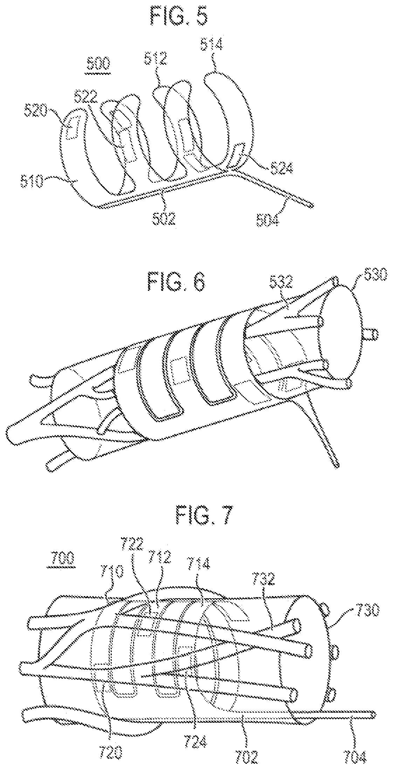

FIGS. 5 and 6 illustrate an additional embodiment of a self-sizing extravascular neural interface 500. The neural interface 500 may be shaped more like a Venus FlyTrap clasp, with a spinal portion 502 connected to a conduit 504 including conductors for the neural interface, and sets of matching portions 510, 512 and 514 extending from the spine 502. The portions 510, 512 and 514 may be substantially orthogonal to the spinal portion 502. Each of the end portions 510 and 512 and the center portion 514 may include an electrode or electrode array 520, 522 and 524, respectively, facing inward so that there may be a good electrical contact between the electrodes and the exterior walls of the target vessel/nerve 530, and allow the artery to pulsate more freely. As previously discussed, this open trench may relieve pressure on the nerves 532 in the target vessel 530 that are sandwiched between the arterial wall and the neural interface 500. The spacing or channels between the portions 510, 512 and 514 may also provide space for the target vessel to pulse and for fluids and nutrients to get to the target vessel.

The electrode or electrode arrays 520, 522 and 524 may also be positioned at different locations within each of the portions 510, 512 and 514. The number of electrodes and their placement with the electrode arrays may vary. As shown in FIGS. 5 and 6, electrode 520 of end portion 510 is positioned near the tip of the end portion 510, electrode 522 of center portion 512 is positioned near the middle of the center portion 512, and electrode 514 of end portion 514 is positioned near the connection point of the end portion 514 to the spinal portion 502. Naturally, different positional configurations (i.e., all electrodes at the tips, middle or spine of the portions, or any other combination of positions) may be possible and particularly selected to provide different circumferential coverage for the type of nerve/vessel and the treatment to be implemented.

As with the neural interface 100 and 300 described above, neural interface 500 is also self-sizing, in that the shapes of the portions 510, 512 and 514 are designed to substantially fit around most of the exterior circumference of the target vessel and the ribs are biased to a relaxed position that will cause them to wrap around most of the target vessel on their own accord once deployed. As used herein, the word "substantially" does not exclude "completely," e.g., a composition which is "substantially free" from Y may be completely free from Y. Where necessary, the word "substantially" may be omitted from the definition of the disclosure. For example, a substantially full turn of a helix may be a full turn of a helix, features positioned substantially opposite may be placed opposite, features spaced a substantially constant distance apart may be spaced a constant distance apart, and electrodes providing a substantially uniform current density may provide a uniform current density.

The portions 510, 512 and 514 may be orthogonal to the spinal portion 502 or at a low helix angel relative the spinal portion 502. The composition of the substrate for the neural interface 500, like neural interfaces 100 and 300, can be silicon or a similar material, and all such neural interfaces can be further treated to prevent early scar formation (i.e., fibrous tissue). Such treatment may be done only on selected surfaces, e.g., the side facing the nerve/arterial wall. For example, silicon may be doped with a steroid drug, such as dexamethasone. The outer surface of the substrate of the neural interface may also, or alternatively, be coated with a hydrophilic polymer, such as poly-2-hydroeyethyl-methacrylate (pHEMA).

The tips of each portion 510, 512, and 514 may be shaped to enable the portions to be gripped by a deployment tool (not shown) for each placement on or removal from a target vessel and/or nerve. Alternatively, attributions, such as attributions 110 and 310 may be added to the exterior surface of the portions 510, 512 and 514 to enable the portions to be pulled back for placement on or removal from a target vessel and released when the neural interface 500.

FIG. 7 illustrates an embodiment of a self-sizing intravascular neural interface 700. As with neural interface 500, neural interface 700 may be shaped like a Venus FlyTrap clasp, with a spinal portion 702 connected to a conduit 704 including conductors for the neural interface, and sets of matching portions 710, 712 and 714 extending from the spinal portion 702. However, in contrast to neural interface 500, each of the portions 710, 712 and 714 may include an electrode or electrode array 720, 722 and 724, respectively, facing outward so that there may be a good electrical contact between the electrodes and the interior walls of the target vessel/nerve 730, and allow the artery to pulsate more freely, which may relieve pressure on the nerves 732 in the target vessel 730 that are sandwiched between the interior arterial wall and the neural interface 700. The spacing or channels (low-pressure trench) between the portions 710, 712 and 714 may also provide space for the target vessel to pulse and unrestricted conduit for fluids and nutrients to get to the interior walls of the target vessel 730, while at the same time, not fully encircling the artery at any point of the cuff geometry. For example, with respect to each embodiment disclosed herein, no portion of the cuff geometry covers a circumference (a complete 360 degree rotation) of a portion of the target vessel orthogonal to any point on the spinal portion.

The electrode or electrode arrays 720, 722 and 724 may also be positioned at different locations within each of the portions 710, 712 and 714. As shown in FIG. 7, electrode 720 of end portion 710 is positioned near the connection point between the spinal portion 702 and the end portion 710, electrode 722 of center portion 712 is positioned near the middle of the center portion 712, and electrode 714 of end portion 714 is also positioned near the spinal portion 702. Naturally, different positional configurations (i.e., all at the tips, middle or spine, or any other combination of positions) may be possible and particularly selected for the range of the circumferential coverage of the nerve/vessel, for the type of nerve/vessel, and for the treatment to be implemented.

In contrast to the extravascular neural interfaces embodiments described above, neural interface 700 may be positioned via a flexible/collapsible catheter (with the neural interface 700 collapsed inside the catheter, not shown) versus an external deployment tools. Depending on the location of the target vessel, the positioning procedure may be minimally invasive. For example, for positioning in a splenic artery, the procedure may be performed through a total percutaneous access via standard (e.g., femoral) artery access. Once the catheter is positioned for deployment of the neural interface 700, the catheter may be withdrawn and the released neural interface will self-size to the inside of the target vessel 730, which requires the portions 710, 712 and 714 to be formed so their normal relaxed position will cause them to fold away from the spine 702 so as to make good contact with the interior walls of the target vessel 730.

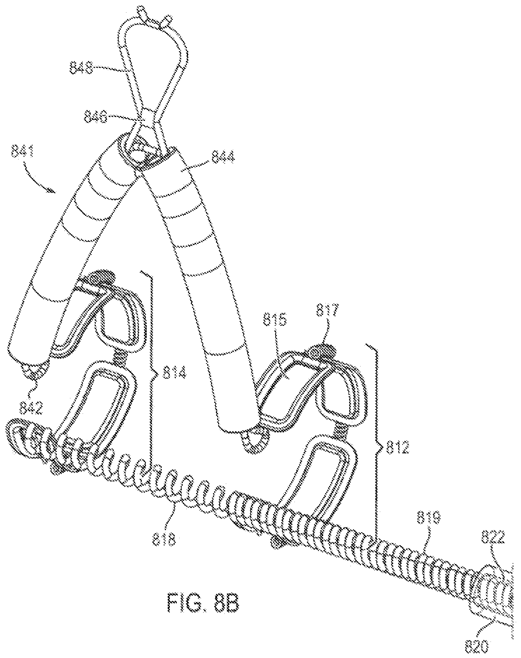

In the embodiment of FIG. 8A, an extravascular bipolar electrode neural interface 800 is illustrated. The interface 800 includes a flexible structure similar to that of FIG. 4B. In FIG. 8B, the neural interface 800 of FIG. 8A is also depicted, but without a flexible substrate 802 and covering for the spinal portion 808, which serves to further illustrate internal components of the neural interface 800 and a deployment tool. The neural interface 800 is similar to the neural interface 400 in FIG. 4B. The flexible substrate 802 may be formed of material similar to that disclosed for neural interface 400. The neural interface 800 may include two arms at either end of the device, such as end portions 804 and 806, which may have open ends 805 and 807, respectively. The end portions 804 and 806 may each be in a C-ring configuration and contain electrode arrays, such as arrays 812 and 814 of FIG. 8B. A center arm portion 830 may be affixed to a spinal portion 808, as are the closed ends of end portions 804 and 806. The center portion 830 may not include any electrodes, serving just to retain the neural interface once positioned, but embodiments may include electrodes.

As shown in FIG. 8B, the four electrodes 815 of each array 812 and 814 are connected in series via three microcoil interconnects 817, which are in turn serially connected to a conductor 818, for array 814, and conductor 819, for array 812. The conductors 818 and 819 may be covered with the same flexible substrate material used to cover the end portions 804 and 806 and central portion 830 over the length of the spinal portion 808 and extending for a short distance from the neural interface 800. Before the conductors 818 and 819 exit the material of the spinal portion they are also covered with a silicon lead body tubing 820 to form the lead body conductor 822.

As previously noted, attributions may be protrusions, but may also be openings or eyelets. As illustrated in FIG. 8A, the attributions may be openings 840 formed at the open end 805 and 807 of end portions 804 and 806. The deployment tool 841 may be comprised of a connector, such as a suture wire 842, grab tab tubing 844, a joiner 846 and a grab tube loop 848. The suture wire 842 may be looped through each opening 840 and through the silicon tubing of grab tab 844. The suture wire 842 may then be brought together at joiner 846 to form a grab tube loop 848. During deployment of the neural interface 800, a surgeon may position the central portion 830 around a target vessel (not shown in FIGS. 8A and 8B), while pulling lightly on the grab tube loop 848. Such pressure will pull the open ends 805 and 807 of end portions 804 and 806 away from the spinal portion 808 and make it possible to position the neural interface 800.

When the neural interface 800 is properly positioned, the pressure may be removed from the grab tube loop 848 so that the open ends 805 and 807 may softly self-size around the target vessel. Although not shown in FIGS. 8A and 8B, the central portion 830 may also include an opening attribution 840 so it may be opened in a similar manner to end portions 804 and 806 for self-sizing around the target vessel. Once the neural interface has been properly positioned, the suture wire 842 may be cut and removed from the openings 840. The opening attributions 840 may be circular holes, oval-shaped slots (not shown in FIGS. 8A and 8B) or other shapes, or eyelets (not shown in FIGS. 8A and 8B) that extend on tabs from the end portions 805 and 807.

In another embodiment, the deployment tool 841 may comprise a further suture wire portion with or without a silicon tubing 844 surrounding the further suture wire portion, where the further suture wire extends between the dual arms of grab tab 844 to form a triangular shape for increased structural stability when deploying the neural interface device 812.

In another embodiment, and referring to FIG. 8C-1, deployment tool 841 comprises a tab-style body 850 instead of the dual arms of grab tab 844 depicted in the embodiment of FIGS. 8A and 8B. In the embodiment of FIG. 8C, a first aperture 852 is analogous to grab tube loop 848, and second and third apertures 854, 856 provide a portion through which a connector such as a suture thread can be anchored to the tab-style body 850. For example, the anchoring may be provided by molding or by use of adhesive material. Once anchored by molding, the apertures 854 and 856 are filled by mold, thus anchoring the connector. Adhesive material could similarly fill the apertures 854 and 856. In some embodiments, the anchoring may be provided without providing any apertures 854 or 856. For example, the connector may be molded when forming the tab-style body 850. In other embodiments, adhesive materials could be used to anchor the connector to at least a part of the tab-style body 850. Tab-style body 850 also can comprise a plurality of sets of eyelets 858 through which the connector is able to pass through. A series of eyelets form a first and second passages through which a connector can pass through as shown in FIG 8C-4. When the connector (e.g. suture thread) passes through the first and second passages formed of the series of eyelets, the connector forms a Y-shape similar to the deployment tool as illustrated in FIGS 8A and 8B. The tab-style body 850 provides increased stability when deploying the neural interface, as the tab keeps the arms of the neural interface parallel (along the edge of the tab where the neural interface is releasably connected to the deployment tab). The planar form of the deployment tab maintains a certain distance between the arms so that the arms are prevented from crossing over or becoming tangled up during deployment.

Other example embodiments of deployment tool 841 are depicted in FIGS. 8C-2 and 8C-3. An example of one of embodiment of deployment tool 841 releasably attached to neural interface device 812 is also shown in FIG. 8C-4.

Tab-style body 850 of deployment tool 841 of FIG. 8C provides advantages in addition to the delivery, positioning and deployment of neural interface 800. Tab-style body 850 provides an increased structural stability when deploying neural interface 800. For example, the two arms releasably attached to neural interface 800 can be stably moved in a single direction.

For example, in some embodiments body 850 can be used as a measuring tool. In one embodiment, and referring to FIGS. 8D-1, 8D-2 and 8D-3, a gap between an end of body 850 and spinal portion 808 having a length L can be measured to determine a degree or amount of stretching of neural interface 800 around a target tissue. This also characterizes a radial length of an electrode arm opening. Understanding these characteristics can be useful to a medical professional user to determine whether an appropriate size of neural interface 841 has been selected for the target tissue. In FIGS. 8D-1, 8D-2 and 8D-3, radial gaps of *L1*, *L2* and *L3*, respectively, are shown and can be assessed by a medical professional user during delivery and deployment of neural interface 800.

In another use of body 850 of deployment tool 841, and referring to FIGS. 8E-1, 8E-2, 8E-3 and 8E-4, the rib-and-groove structure of body 850 can be used as a "measuring tape"-style measurement tool in some embodiments. The rib-and-groove structure of body 850 can be flexible such that it at least partially conforms around the target tissue, and in doing so it provides another way in which a medical professional user can use body 850 to assess size and fit of neural interface 800 relative to a target tissue. This can be accomplished in several ways. In one embodiment, information can be provided to a medical professional user that translates a number of ribs (or grooves) into usable information. For example, a separate of 3-5 ribs is acceptable, while 2 or fewer means the cuff is too large and 6 or more means it is too small. Thus, simply counting the ribs (or grooves) can directly provide fit information, as illustrated in FIGS. 8E-1, 8E-2 and 8E 3. In another embodiment, a user, such as a medical professional can first count ribs (or grooves) as shown in FIGS. 8E-1, 8E-2 and 8E-3 and use known measurements between adjacent ribs (or grooves) to assess size and fit, as shown in 8E-4. Similarly, the known measurements can be translated into a table showing cuff opening as a fraction of circumference and advising medical professional users as to which are suitable or not. In the table of 8E-4, the third through seventh values are suitable, the first two indicate the cuff is too large, and the last two that the cuff is too small for the target tissue. Depending on the target and the specific neural interface embodiment used, a table with different predetermined values may be used.

As previously noted, tab-style body 850 also comprises a plurality of sets of eyelets 858. In use, a suture wire may be looped through each eyelet 858 and then brought together at first aperture 852 to form a grab loop. During deployment of the neural interface 800, a surgeon may position neural interface 800 around a target vessel while pulling lightly on the grab loop. Such pressure will pull the open ends 805 and 807 of end portions 804 and 806 of neural interface 800 away from the spinal portion 808 and make it possible to position the neural interface 800 where desired.

When neural interface 800 is properly positioned, the pressure may be removed from the grab loop so that the open ends 805 and 807 may gently self-size around the target vessel. Once the neural interface has been properly positioned, the suture wire may be cut and removed from the eyelets 858 and first aperture 852. The first aperture 852, second and third apertures 854, 856 and eyelets 858 may be circular holes, oval-shaped or oblong slots, or other shapes, or features that extend on tabs from the end portions 805 and 807 of neural interface 800.

The deployment tool 841 with a tab-style body (also referred to as a deployment tab) may comprise a thickness and/or width slightly larger than the thickness and/or width of the neural cuff. The deployment tab may include an anchored suture that is wound through the deployment tab and removably attached to the neural cuff (for example by a connector such as a suture thread at a deployment attribute of the neural cuff such as an opening at an open end of the arm). A cut through at least a portion of the deployment tab may completely detach the deployment tool from the neural cuff. The deployment tab may include a series of transverse (or lateral, along a width of the deployment tab) ridges and valleys on one side, which may serve as a cut through guide and may enable the deployment tab to be rolled into a small size for delivery. The deployment tab may include a series of longitudinal ridges and valleys on the opposite side as shown in FIGS. 8C-1 and 8C-2, for example on the side shown in FIGS. 8D-1, which may serve to minimize contact surfaces (including when the deployment tab is rolled up and with tissue during deployment). The deployment tab may include a tapered proximal end and be configured to operate as an instrument to check the dissection opening is large enough for the cuff (e.g. a go/no-go gauge) as well as a blunt dissection tool. If a thickness and/or width of the deployment tab will not fit through the dissection, a slightly smaller neural cuff may not fit. The anchored suture is positioned within the deployment tab so that when at least a portion of the deployment tab is cut through, the suture is cut, thereby releasing the deployment tab from the pre-attached portion of the neural cuff.

Other tools and accessories may be provided to assist a surgeon in delivering, positioning, and deploying embodiments of neural interfaces discussed herein. For example, FIGS. 8F-1, 8F-2 and 8F-3 depict a lead cap device 860. Lead cap device 860 in arranged on and protects an end of the lead body during delivery and deployment of the neural interface, during which stresses including implantation loads and mechanical interactions with surgical tools (such as graspers) on the lead body as it is pushed and pulled into place could cause damage to the lead body or the conductors. Lead cap device 860 is sized and configured such that it can fit into the cannula or a catheter. For example, in one embodiment lead cap device 860 is sized to fit into a 5 mm cannula, though lead cap device 860 can be provided in a range of sizes such that it can be compatible with a range of catheter/cannula sizes.

In the embodiment of FIGS. 8F-1, 8F-2 and 8F-3, lead cap device 860 comprises a body 862, a setscrew block 864 comprising a set screw 866, and a suture loop 868.

In one embodiment, body 862 comprises a transparent or semi-transparent biocompatible material, such as silicone. Such a material enables visual feedback during use, as a surgeon can see into body 862 to determine how far an end of lead body 917 has gone into an internal cavity 870 of body 862. In some embodiments only a portion of body 862 may be transparent.

Internal cavity 870 includes a retention constriction 872, shown in the cross-sectional view of 8F-2. Internal cavity 870 also passes through setscrew block 864. This configuration allows for a lead body 917 (see 8F-3) to be fed into a first end 874 and internal cavity 870 of lead cap device 860. Once fully inserted and arranged within internal cavity 870 and setscrew block 864, setscrew 866 can be tightened to retain lead body 917 therewithin. This tightening of setscrew 866 can be accomplished by a torque wrench (not depicted). The torque wrench may give an audible click when maximum or desired torque has been applied. Setscrew block 864 is formed to engage with body 862 such that rotation, movement or misalignment of setscrew block 864 with respect to body 862 is prevented when setscrew 866 is tightened and lead cap device 860 is manipulated during routing.

In wired embodiments, lead body 917, more specifically an IPG connector part of the lead body 917, should be inserted sufficiently into lead cap device 860 that the portion of lead body 917 that engages with set screw 866 does not contain any sensitive components of lead body 917, such as contact portions of the lead conductors themselves. Damage to these contact portions during implantation could impair electrical isolation properties where the lead body 917 interfaces with a pulse generator, such as an implantable pulse generator (IPG). In other words, the end portions of lead body 917 configured to be coupled with other system components for use, such as the pulse generator, should be advanced past set screw 866 towards a second end 876 of lead cap device 860 and suture loop 868. When so positioned, retention constriction 872 also functions to retain lead body 917 therewithin and in some embodiments can do so even if setscrew 866 is not tightened (or is not sufficiently tightened).

The configuration and features of lead cap device 860 enable a surgeon to push and pull on lead body 917, in any orientation, in order to route it into position. Suture loop 868 can be grasped using a grasper tool or other device in order to pull lead cap device 860 (and thereby lead body 917). The portion of body 862 proximate second end 876 also can be grasped and pulled during routing. Similarly, the tapered configuration of first end 874 of body 862 can be pushed during routing.

Though discussed and described with respect to particular examples (such as neural interface 800), tab-style body 850, lead cap device 860, and other accessories and techniques described above also are applicable to and can be used with other embodiments of neural interfaces. Furthermore, not all embodiments necessarily include lead bodies. For example, the neural interface 900 of FIG. 9A described in more detail below is shown as wired to an implantable pulse generator, but it should be understood that the neural interface device 900 could instead by powered wirelessly by including a receiver or coil at the neural interface device 900 instead of a lead body 917 that provides a hard-wired connection. In some embodiments, the implantable pulse generator referred to herein need not be implanted, if the neural interface device 900 can be powered by a wireless pulse generator such as a device worn by a user. In some other embodiments, the neural interface 900 may comprise a miniature implantable pulse generator (IPG) with wireless antenna for receiving power and communication from a transmitter. The IPG may receive power from an external source, and/or may comprise a battery for being charged from an external source, wherein the IPG is powered by said battery or an external source. While the following figures refer to wired lead-body based embodiments, it should be understood that these embodiments could alternatively be wireless, and that pulse generators described herein need not be implanted or even implantable unless specified otherwise.

For example, FIG. 9A illustrates another embodiment of a neural interface 900 in accordance with the present disclosure. Neural interface 900 may be similar to neural interfaces 100, 200, 300, 400 discussed herein above unless otherwise mentioned herein. For example, neural interface 900 may be formed of the same or a similar flexible substrate of material (i.e., silicone) and share other features.

Neural interface 900 comprises a spinal portion 902, a first C-ring portion 904, a second C-ring portion 906, and a third C-ring portion 908. Spinal portion 902 comprises a first end 901 coupled to a lead body 917 comprising a conductor 918, and a second end 903 at least partially coupled to first C-ring portion 904. At least a portion of conductor 918 extends from lead body 917 and within spinal portion 902 from first end 901 towards second end 903, terminating in a connection to first C-ring portion 904. At an opposite end, lead body 917 and conductor 918 are connectable via a connector to an implantable pulse generator (not depicted).

Lead body 917 comprises conductor 918, which in one embodiment is a coradial bifilar conductor. The coradial bifilar design of conductor 918 provides increased flexibility and in some embodiments may make conductor 918 stretchable. In other embodiments, if the tube covering the coradial bifilar conductor 918 is not stretchable, the lead body has increased flexibility but not stretchable. These characteristics provide increased decoupling between lead body 917 and spinal portion 902. This means that even if lead body 917 is moved or flexed during application, spinal portion 902 (and C-ring portions 904, 906, 908) will not be moved on or off of the target tissue. Additionally, the coradial feature of conductor 908 makes conductor 908 more crush-resistant, which can be a benefit during laparoscopic delivery of neural interface 900 to target tissue while still maintaining flexibility to aid in delivery and placement.

In other embodiments, one or both of lead body 917 and conductor 918 can comprise structures or configurations to provide strain relief. Referring also to FIGS. 9B and 9C, in some embodiments lead body 917 can comprise strain-relieving undulating sections 917b intermittently located between linear sections 917a. Any particular lead body 917 can comprise one undulating section 917b or a plurality of undulating sections 917b, and the particular configuration of the undulating section(s) 917b can vary. Undulating sections 917a help in breaking up or interrupting large or strong motions affecting lead body 917 into smaller, discrete or localized weaker movements.

Two examples of undulating sections 917b are depicted in FIGS. 9B and 9C, but these examples are not limiting with respect to all of the possible embodiments contemplated by this disclosure. For example, the undulations can be sinusoidal, square, rectangular, helical, coiled, regular, irregular, or other shapes or combinations of shapes. The number of undulations also can vary, with some undulating sections 917b having more or fewer undulations, as may be desired or preferred for areas that experience more or less strain in use. In general, however, each turn in an undulating pattern prevents pressure waves from migrating longer distances along the length of lead body 917.

In some embodiments, undulating sections 917b can be located near neural interface 900, while in other embodiments undulating sections 917b can be located away from neural interface 900 or at various points along the length of lead body 917. Undulating sections 917b near neural interface 900 can help to block displacement forces from reaching neural interface 900 and affecting its stability and placement.

In still other embodiments, lead body 917 also can comprise at least one anchoring sleeve or tab 919. Though the configuration can vary, the term "anchoring tab" generally will be used herein even though in some embodiments the anchoring structure can comprise a sleeve or other device. Anchoring tabs 919 can be located at one or more points along lead body 917 and can be used to secure lead body 917 to tissue, such as by suturing anchoring tab 919 to tissue. For example, fixation of a lead anchor to a crus of the diaphragm can be accomplished with one or two permanent sutures. The right crus can be reached by retracting the lateral segment of the left lobe of the liver, such as with a Nathanson retractor, to allow visualization of the right crus of the diaphragm. The anchor of the lead can be placed in proximity to the right crus of the diaphragm and affixed to the right crus using one or two permanent sutures.

In one embodiment depicted in FIG. 9D, an anchoring tab 919 is located proximate first end 901 of spinal portion 902. In this embodiment, lead body 917 is about 650 mm long, and anchoring tab 919 is coupled to lead body 917 about 200 mm from first end 901 of spinal portion 902. Anchoring tab 919 is about 10 mm square. These dimensions are examples of only one embodiment and can vary proportionately or otherwise in other embodiments. In some embodiments, an anchoring tab 919 is located proximate one or more undulating sections 917b, while in one particular embodiment an anchoring tab 919 is located on each side of an undulating section 917b.

Anchoring tabs 919 can be coupled to lead body 917 in a variety of ways. As mentioned above, in some embodiments anchoring tabs 919 comprise sleeves that extend around lead body 917 and may be slidable along at least a portion of lead body 917 (e.g., between adjacent undulating sections 917b). In other embodiments, these sliding anchoring tabs 919 may comprise a fastener, such that the sliding anchoring tab 919 can be placed in a desired location along the lead body 917 and then fixed by the fastener, which may include means to tighten around the lead body 917, or suture or silicone adhesive to be fixedly attached to the desired location along the lead body 917. In still other embodiments, anchoring tabs 919 are fixedly coupled to a particular point along lead body 917, such as by being adhered to lead body 917 by a silicone adhesive near first end 910 of neural spinal portion 902 of neural interface 900.

Anchoring tabs 919 can comprise many different biocompatible materials. In one embodiment depicted in FIG. 9D, anchoring tabs 919 comprise a mesh material, such as a coated mesh material. For example, anchoring tabs 919 can comprise a polyethylene terephthalate (commercially known as DACRON) mesh material coated with a room-temperature vulcanization cure silicone dispersion Nusil MED-6605. The mesh itself can comprise a warp knit multifilament structure with 140 Denier, a thickness between about 0.4 mm and about 0.6 mm (such as about 0.5 mm in one example embodiment), and a pore size of about 0.9 mm, 1 mm, 1.1 mm, or larger or smaller. In some embodiments the pores are not round and have an ovular, oblong, or other shape with a size of about 1.0 mm by about 1.1 mm. These dimensions can vary, such as by plus/minus 5 percent, plus/minus 10 percent, plus/minus 15 percent, plus minus 20 percent, plus/minus 25 percent, plus/minus 30 percent, plus/minus 35 percent, plus/minus 40 percent, plus/minus 45 percent, or plus/minus 50 percent in other embodiments.

The coated mesh structure of anchoring tabs 919 can provide a variety of advantages. First, the mesh can maximize resistance to tearing. Coating the mesh structure can minimize tissue ingrowth by partially or completely filling the pores of the mesh, thereby reducing or preventing growth of tissue into the pores of the mesh over time, which aids in explantability of anchoring tabs 919 and neural interface 900 overall and reduces the likelihood of serious complications that can result from ingrowth of tissue. The coated mesh structure of anchoring tabs 919 also helps to minimize or reduce the rigidity of anchoring tabs 919, thereby improving reliability (as the smoother the stiffness gradient is at the transition from lead body 917 to anchoring structure 919, the more reliable the junction will be, generally speaking). Minimizing or reducing rigidity also aids surgical implantation, as the less rigid anchoring tab 919 is, the easier it is to suture in place. Additionally, the coated mesh structure helps to maximize or increase adhesion between anchoring structures 919 and lead body 917. In embodiments in which leady body 917 comprises silicone, the silicone adhesive and silicon coating of the mesh of anchoring structure 919 provide a strong bond to affix the anchor in the desired position, such as the right or left crus of the diaphragm, to hold the lead securely in place and avoid disruption of implant operation due to lead travel.

First end 901 of spinal portion 902 defines a tapered portion that tapers from a maximum circumference to a minimum circumference. In the embodiment of FIG. 9A, the maximum circumference occurs at a point proximate the C-ring portions 904, 906, 908, in particular where spinal portion 902 is at least partially coupled to third C-ring portion 908. The minimum circumference occurs where spinal portion 902 terminates along lead body 917. The length and dimensions of the tapered portion of first end 901 provide the benefits of reducing the stiffness gradient in transitioning from the relatively stiff spinal portion 902 to the relatively flexible lead body 917. Large stiffness gradients lead to poor flex fatigue performance and may cause rupture of conductor 918 at the transition. Advantageously, embodiments of neural interface 900 provide a smoother transition in stiffness/flexibility, which can improve structural stability at the coupling point of spinal portion 902 and lead body 917. At the same time, the tapering of first end 901 helps to improve flexibility of this portion of neural interface 900 by providing sufficient flexibility to enable positioning, placement and deployment of neural interface 900 and each of first C-ring portion 904, second C-ring portion 906, and third C-ring portion 908 and to maintain a satisfactory level of comfort once deployed. In various embodiments, the tapered portion is about 2 mm to about 5 mm long, such as about 2.1 mm in one example embodiment. The circumference of the tapered portion can taper from about 3 mm to about 1.5 mm, such as from about 2.5 mm to about 1.75 mm in one example embodiment. The tapering angle can range from about 5 degrees to about 15 degrees, such as about 10 degrees in one example embodiment.

Second end 903 presents an angled, blunted, or rounded-off surface, in that spinal portion 902 extends to an outer edge of first C-ring portion 904 at a bottom or lower side (with respect to the orientation of FIG. 9A on the page) but terminates further back on a top or upper side. In other words, spinal portion 902 has a substantially circular cross-section, and a plane parallel to the circular cross-section is at an angle of greater than 0 degrees and less than 90 degrees with respect to the angled surface of the second end. This surface can be substantially flat, curved, or contain both flat and curved portions. For example, in the embodiment depicted in FIG. 9A the surface is substantially flat from the top or upper end until proximate first C-ring portion 904, where the surface curves downward to first C-ring portion 904. The angle, degree of curvature, relative composition of flat and curved portions, and other characteristics of this end surface can vary from the example depicted in FIG. 9A. In general, however, second end 903 includes such an end surface to eliminate a possible pressure point when neural interface 900 is deployed. This can improve patient comfort and may also increase maneuverability and placement of neural interface 900 during the deployment process.

Between first end 901 and second end 903, one end of each of first C-ring portion 904, second C-ring portion 906, and third C-ring portion 908 is coupled to spinal portion 902. In the embodiment depicted in FIG. 9A, first C-ring portion 904 and third C-ring portion 908 are coupled to spinal portion 902 in the same orientation, with the opening in each C-ring portion 904, 908 on the back or left side of neural interface 900 with respect to its orientation on the page. C-ring portion 906 is coupled to spinal portion 902 in an opposing orientation, with the opening in C-ring portion 906 on the front or right side of neural interface 900 with respect to its orientation on the page. In other words, first C-ring portion 904 and third C-ring portion 908 extend from spinal portion 902 in a direction opposing a direction of second C-ring portion 906.

This relative arrangement of C-ring portions 904, 906 and 904 enables C-ring portions 904 and 908 to remain static (or move) together, while C-ring portion 906 moves (or remains static), during deployment of neural interface 900. As such, neural interface 900 provides a modest overall profile, enabling laparoscopic (i.e., minimally invasive) delivery while at the same time providing sufficient flexibility and relative movement of C-ring portions 904 and 908 with respect to C-ring portion 906 to enable neural interface to be "opened" for extravascular placement and deployment. This configuration of C-ring portions 904, 906 and 908 also increases the likelihood that neural interface 900 will not open unexpectedly after deployment and move to an undesired position.

In some embodiments, each C-ring portion 904, 906, 908 may have a very low helix angle, i.e., pitch, relative to the spinal portion 902, which enables neural interface 902 to be helical but still have a significantly shorter length. The helix angle may be between approximately 15 and 30 degrees but may also be less than 15 degrees.

In other embodiments, each C-ring portion 904, 906, 908 may not be helical or have a helix angle, i.e., pitch, relative to the spinal portion 902, for example as illustrated in Figure 9A and Figures 13A, 13B and 13C. Additionally, each C-ring portion 904, 906, 908 comprises rounded or smooth edges and ends, which can ease delivery of neural interface 900, reduce damage to adjacent tissues, and increase comfort to the patient. In other embodiments not specifically depicted, neural interface 900 may comprise more or fewer C-ring portions; C-ring portions with the same coupling and opening orientations, alternating coupling and opening orientations, or other patterns of C-ring portion coupling and C-ring portion orientations; C-rings of different relative sizes; C-ring portions of different or varying helical angles; and other variations, including as discussed herein with respect to other embodiments.

In the embodiment depicted in FIG. 9A, each C-ring portion 904, 906, 908 of neural interface 900 has a substantially regular or even thickness along its length. In other words, the thickness of each C-ring portion 904, 906, 908 from a first end coupled to spinal portion 902 to a second end is approximately the same, not accounting for any thickness added by electrodes on any C-ring portion. The thickness of each C-ring portion 904, 906, 908 alternatively can be expressed in a ratio of cuff inner diameter D (see FIG. 10A) to C-ring portion thickness. For example, if the diameter D of a C-ring portion 904, 906, 908 is 6 mm, an example thickness of each C-ring portion 904, 906, 908 can be 1 mm, for a ratio of diameter to thickness of 6:1. In another example, if the diameter D of a C-ring portion 904, 906, 908 is 7 mm, an example thickness of each C-ring portion 904, 906, 908 can be 1.3 mm, for a ratio of 5.4: 1. In yet another example, if the diameter D of a C-ring portion 904, 906, 908 is 9 mm, an example thickness of each C-ring portion 904, 906, 908 can be 1.6 mm, for a ratio of 5.6:1. Thus, generally speaking, a ratio of cuff inner diameter to C-ring portion thickness can range from about 5:1 to about 7:1, such as about 5.3:1 to about 6.5:1, or about 5.4:1 to about 6.2:1, or about 5.5:1 to about 6:1, or about 5.6:1 to about 6:1, in various embodiments.

As can be seen from these examples, the thickness of the C-ring portions 904, 906, 908 increases as the diameter increases, which can provide a similar pressure regardless of the diameter of the cuff. Those skilled in the art will appreciate that without adjusting the thickness with diameter, the pressure would be expected to decrease as the diameter increases. Those skilled in the art also will recognize that the thickness will depend on properties (e.g., stiffness) of the material used to form C-ring portions 904, 906, 908, which means that the ratios above (associated with silicone) may vary according to the properties of selected materials in other embodiments. Additionally, the ratios can depend on the aspect ratio of the C-ring portions, the aspect ratio of the electrodes on the C-ring portions, the number of electrodes on the C-ring portions, the materials used for the electrodes, and other factors. Expressed another way, embodiments of neural interface 900 can be configured to apply (or maintain) pressure to target tissue within the C-ring portions in a range of about 0 mmHg to about 30 mmHg, such as about 0 mmHg to about 25 mmHg, or about 0 mmHg to about 20 mmHg, or about 0 mmHg to about 15 mmHg, or about 0 mmHg to about 10 mmHg, or about 0 mmHg to about 5 mmHg, or about 0 mmHg to about 2 mmHg or about 5 mmHg to about 20 mmHg, or about 5 mmHg to about 10 mmHg, for example about 20 mmHg, or for example about 10 mmHg, or for example about 5mmHg. This pressure can be measured at a variety of points along the inner diameter of neural interface 900 and can be an average value, a mean value, or a median value of a plurality of values taken at a plurality of points, or particular value at a particular point.

In other embodiments, thickness can vary along the length of the C-ring portion, providing another way for uniform pressure to be provided along the length of each C-ring portion (i.e., at each electrode). Referring, for example, to FIGS. 10A-10C, end views of a neural interface 1000 are depicted. In FIG. 10A, a thickness of a C-ring portion 1010 varies from a first thickness *T1* at a first end coupled to spinal portion 1002, to a second thickness *T2* at point opposite spinal portion 1002, and then to a third thickness *T3* at a second end. In the embodiment depicted, thicknesses *T1* and *T3* are similar or the same, and thickness *T2* is the maximum or greatest thickness of C-ring portion 1010.

In one example embodiment, the thickest part of a C-ring portion (e.g., at *T2* in FIG. 10A) is approximately twice the thickness of the ends of the C-ring portion (e.g., *T1* and *T3* in FIG. 10A). Additionally, the thickness of the C-ring portion between the electrodes can be important. In one particular example, a 7 mm neural interface has gaps between adjacent electrodes at 31.5 degrees and 94.5 degrees from the center of the "C," and the thickness of the gaps at these angles corresponds to 1.34 mm and 0.95 mm. This results in a ratio of 1.4 to 1.

In other embodiments, the thickness may vary in other ways along a length of any C-ring portion. For example, in FIGS. 10B and 10C two different examples of localized thinning of a C-ring portion 1010 are depicted. In other examples, the thickness of any individual C-ring portion of a particular neural interface, such as neural interface 1000, can vary with respect to the thickness of other C-ring portions of the same neural interface 1000. For example, the thickness of first and third C-ring portions may vary as depicted in FIGS. 10A-10C while the thickness of a middle second C-ring portion may remain constant, in particular if it does not comprise an electrode array (such as for C-ring portion 906 in neural interface 900 depicted in FIG. 9).

In general, however, the objective is to reduce the contact pressure for the electrode 1012 nearest spinal portion 1002 and the electrode 1012 at the far (open) end of the C-ring portion 1010. In C-ring portions with constant thickness, these two electrodes will bear most of the load. The tapered embodiment of FIG. 10A can achieve this by reducing the beam thickness of the C-ring portions that connect the two "outer" electrodes to the electrode in the middle. Similar advantages with respect to varying thickness and pressure management may be seen for C-ring portions without electrode arrays as well.

Referring again to FIG. 9A, and as with neural interfaces 100, 200, 300 and 400, each C-ring portion 904, 906, 908 of neural interface 900 may include one or more electrodes or an array of electrodes 912. Each electrode array 912 is electrically coupled to a conductor 918 that extends into spinal portion 902. One electrode of each electrode array 912 is coupled to conductor 918 through spinal portion 902 via other electrodes.

The electrode of each electrode array 912 that is coupled to conductor 918 can be coupled thereto in a variety of ways. In one embodiment, this coupling is accomplished by welding, such as by laser-welding. The particular configuration of the laser weld can provide strain relief, reducing the likelihood that relative movement of the C-ring portions 904, 906, 908 with respect to conductor 918 will cause separation or breakage of the weld. In conventional arrangements, the wire of conductor 918 would be welded to the electrode in a substantially perpendicular orientation, as shown in FIG. 9F-1. In embodiments of this disclosure, in contrast, the wire of conductor 918 is welded to the electrode at an angle or tangentially. This angle provides strain relief in the coupling of the electrodes to the conductors as the conductors are not required to flex or turn as abruptly at the weld point. This configuration also provides more space and surface area for the weld coupling, as the tangential angles of weld can increase surface area for welding.

In the embodiment of FIG. 9A, the electrode array 912 of each of C-ring portion 904 and C-ring portion 908 comprises four electrodes. A first electrode is arranged at the end of each C-ring portion 904, 908 that is coupled to spinal portion 902 and electrically coupled to conductor 918 by a conductor wire 920. A second electrode is arranged adjacent to the first electrode and electrically coupled to the first electrode (and thereby to the conductor 918) by an inter-electrode coil 922, which for example may be a micro-coil, a stranded cable or a metal ribbon such as a platinum metal. One example of such a ribbon is shown in FIG. 15I, for example, described in more detail below. In other embodiments, the electrodes may be formed of a unitary body, for example as in the embodiments shown in FIG. 15A-H. A third electrode is arranged adjacent to the second electrode, on an opposite side of the second electrode to the first electrode, and electrically coupled to the second electrode (and thereby to the conductor 918) by another inter-electrode coil 922. A fourth electrode is arranged between the third electrode and the open end of the C-ring portion and electrically coupled to the third electrode (and thereby to the conductor 918) by another inter-electrode coil 922.

The individual electrodes of electrode arrays 912 of neural interface 900 may be evenly spaced within C-ring portions 904, 906, 908. By evenly spacing the electrodes on C-ring portions 904, 906, 908, the inter-electrode distance is more constant, which may provide a more uniform current density distribution and enhancement of the effectiveness of neural interface 900. In some embodiments, the position of the electrodes in electrode arrays 912 may be staggered in order to achieve better, or different electrical coverage. Certain characteristics of neural interface 900 may each be chosen for a particular application of neural interface 900, such as: the spacing between adjacent C-ring portions 904, 906, 908; the spacing between electrode arrays 912; the spacing between electrodes of electrode arrays 912; the size and shape of the electrodes; the size, shape, and number of electrodes in electrode arrays 912; the inter-electrode distance within electrode arrays 912; and the helix angle. For example, utilization of neural interface 900 for treatment by utilizing a target, for example the splenic artery, may require different characteristics than utilization of neural interface 900 for treatment of a different vessel. For instance, when utilized for splenic artery treatment (e.g. treatment provided by the neural interface being provided around the splenic artery) an electrode width of approximately 1 mm to approximately 4 mm, such as width ranges between approximately 1mm and approximately 2 mm or between approximately 2mm and approximately 3 mm, may be appropriate. When utilized for treatment via different vessels, different electrode widths may be desirable.

Electrode coils 922 can be configured to provide electrical couplings between adjacent electrodes of electrode arrays 912 while also providing desired flexibility themselves and at the same time not inhibiting flexibility or conformity of C-ring portions 904, 908. Flexibility can be provided by the coiled arrangement of electrode coils 922, as coils provide flexibility with spring properties that straight conductor wires do not have. For example, electrode coils 922 can have improved flex fatigue performance as compared with straight wires. In use, neural interface 900 sits on a pulsatile structure such that electrode coils 922 will be subjected to multitudes of small flex loads. Coiled electrical couplings have better flex fatigue performance than straight wires. Similarly, conformity of C-ring portions 904 and 908 can be retained or enhanced by adjusting the diameter and pitch of electrode coils 922. In example embodiments, a coil pitch of electrode coils 922 can be in a range of 0.05 mm to 0.3 mm, such as in a range of 0.10 mm to 0.25 mm, for example 0.10 mm, 0.15 mm, or 0.23 mm. A wire diameter of electrode coils 922 can be in a range of 0.05 mm to 0.10 mm, such as a range of 0.07 mm to 0.09 mm, for example 0.076 mm or 0.081 mm, in various example embodiments. A coil diameter of electrode coils 922 can be in a range of 0.2 mm to 0.6 mm, such as in a range of 0.3 mm to 0.5 mm, for example 0.38 mm, 0.43 mm, or 0.46 mm. In various embodiments, these dimensions can be selected from example ranges according to a determined relationship between any of these dimensions or other dimensions or characteristics of the electrodes, the C-ring portion, or the overall neural interface.

In the embodiment depicted in FIG. 9A, there are no electrodes arranged on second C-ring portion 906 and the same number and arrangement of electrodes in electrode arrays 912 on first and third C-ring portions 904 and 908. In other embodiments, the number and arrangement of electrodes or electrode arrays 912 on any individual C-ring portion 904, 906, 908 can vary, with more or fewer electrode arrays 912 used overall or more or fewer electrodes arranged on any particular C-ring portion 904, 906, 908. Electrodes may be arranged on one C-ring, some but not all C-ring portions, or all C-ring portion s 904, 906, 908.

In some embodiments, multiple electrodes or an electrode array 912 on any one C-ring portion, such as are depicted on C-ring portions 904 and 908 in FIG. 9A, may be considered to be a single electrode. In other words, in some contexts the embodiment of neural interface 900 depicted in FIG. 9A comprises two electrodes, one on C-ring portion 904 and one on C-ring portion 908, with each electrode comprising multiple (four) electrodes portions.

The electrode may be very thin (for example between 25 µm and 50 µm) but not so thin so as to make interconnection (which may be accomplished by, e.g., laser welding) to the electrode difficult. In some embodiments the electrodes may be recessed or embedded into their respective C-ring portions, with silicone rims or silicone webbing used to hold the electrodes in place. In other embodiments, "segmented" electrode designs may provide better mechanical compliance, create the possibility of surface features, i.e., protruding electrodes, and make it possible to control each electrode individually (i.e., current steering). Segmented electrodes provide increased flexibility to the neural interface, thereby making it possible open a C-ring portion with a deployment tool wider and for a longer period of time, without creating excessive stress on the electrodes, than might be possible with a single electrode.

In still other embodiments, the electrodes can be configured with or comprise features to improve flexibility, prevent delamination of the electrodes from the C-ring portions, and otherwise enhance interoperability between the electrodes and the C-ring portions. For example, in embodiments in which the electrodes are recessed or embedded into their respective C-ring portions, the electrodes can comprise or be coupled with an electrode pad such that it is the electrode pad that is recessed or embedded into the C-ring portions. The electrode pad can comprise the same material as the electrode or a different material, such as a material with desired properties for bonding or coupling the electrode to the C-ring portion. The material of such an electrode pad can vary in embodiments and may be selected according to the materials of the electrode (such as platinum) and C-ring portion (such as silicone).

Additionally or alternatively, the portion of the C-ring portion into which the electrode (or electrode pad is embedded can be slightly larger than the electrode or electrode pad to allow for curvature and movement of the electrode or electrode pad as the neural interface is deployed (i.e., when the C-ring portions experience the most significant deformation) while maintaining the electrodes and electrode pads in desired positions after deployment. For example, a gap can be provided in the C-ring portion on one or both ends of the electrode or electrode pad, where the ends in FIG. 9A are the two sides of the electrode 912 that are shorter. In other words, a length of a recess into which the electrode or electrode pad is placed in the C-ring portion is longer than a length of the electrode or electrode pad itself which is protruding and act as an exposed surface or contacting surface (for contacting with the target).

Characteristics of the recess also can be selected to accommodate curvature and movement of the electrode or electrode pad therein. For example, the overall shape of the recess can be the same or different from the electrode or electrode pad. In the embodiment of FIG. 9A, the electrode is rectangular with rounded corners, and a recess in the C-ring portion into which such an electrode is placed also can be rectangular with rounded corners, or it can be rectangular with square corners or have some other shape that is different from that of the electrode (or electrode pad) itself. In these or other embodiments, the electrode or electrode pad also can comprise one or more flanges or anchors configured to fit within or otherwise engage the recess in the C-ring portion and retain the electrode or electrode pad therein.

Additionally or optionally, the electrode may comprise various different materials in order to achieve a desired properties, for example flexibility or electrical charge injection properties. For example, the electrodes may comprise platinum, or be formed from an alloy of platinum and iridium, for example an alloy made from 90% platinum and 10% iridium. Alternatively or additionally, the surfaces of the contact electrodes may be coated possibly with PEDOT, TiNi, IrOx, PtBlack or treated using a process of laser roughening.

In still other embodiments, and in addition to or instead of other electrode and electrode pad features discussed herein, each electrode can comprise a flange having one or more perforations. These perforations can improve the mechanical coupling between the electrode and the C-ring portion, prevent delamination of the electrode from the C-ring portion, increase the flexibility of both the electrode and the C-ring portion (particularly during placement and deployment of the neural interface), and provide other benefits appreciated by those having skill in the art.

For example, FIG. 11A is a partial view of FIG. 9A and depicts an electrode 930 comprising an electrode contact 932 and an electrode flange 934. Electrode flange 934 comprises at least one perforation 93 6A. In the embodiment depicted, electrode flange 934 comprises six perforations 936A, but in other embodiments more or fewer perforations can be included. Perforations 936A are arranged on each longer side of electrode flange 934, with three perforations 936A on one side and three perforations 936A on the other, opposing side. Each perforation 936A is rectangular with rounded corners or rounded shorter ends. In other embodiments, perforations 936A can be arranged on the shorter sides, on both the shorter and longer sides, or in some other configuration. While perforations 936A are equally sized and evenly spaced, in other embodiments the size, shape, spacing, placement, orientation, or other characteristics of perforations 936A can vary.

For example, in the embodiment of FIG. 11B, electrode flange 934 again comprises six perforations 938B, though perforations 938B are round or circular, with three arranged on one shorter end of electrode flange 934 and three arranged on the other shorter end of electrode flange 934. Electrode flange 934 in FIG. 11B also has a more rounded periphery than in the embodiment of FIG. 11A.

The embodiment depicted in FIG. 11C is similar to that of FIG. 11B, though in this embodiment there are two perforations 936C, both generally rectangular though with rounded shorter ends. One perforation 936C is arranged on each shorter end of electrode flange 934.

The embodiment of FIG. 11D is similar to the embodiment of FIG. 11C, except that electrode flange 934 is larger with respect to electrode contact 932 and wider, such that its corners are rounded but not its shorter ends. Additionally, perforations 936D also are larger, with lengths similar to electrode contact 932 and widths larger than those of perforations 936C in FIG. 11C.

FIG. 11E depicts an electrode 930 similar to that of FIG. 11D except that it comprises four perforations 936E. Each perforation 936E is a square with rounded corners and is arranged in each corner of electrode flange 934.

Yet another embodiment is depicted in FIG. 11F. In this embodiment, perforations 936F comprise cutouts or apertures along the periphery of electrode flange 934. In other words, perforations 936F form notches along the long edges of electrode flange 934.

In FIG. 11G, electrode flange 934 extends along a central portion of each longer edge of electrode contact 932. The electrode flange 934 also comprises a curved under edge portion 937 for increased mechanical connection between the electrode and the insulating portion or the insulating portion of the neural interface. This curved under edge portion may also provide an area for interconnection to form a mechanical connection (e.g. for welding). Two perforations 936G are formed along the length of electrode flange 934 on each side.

The embodiment of FIG. 11H is similar to the embodiment of FIG. 11G but omits perforations altogether.

The embodiment of FIG. 11I also is similar to the embodiment of FIGS. 11G and 11H except that, as compared with the embodiment of FIG. 11G, it additionally includes portions of electrode flange 934 on each shorter end of electrode contact 932, and these portions each include a round or circular perforation 936I. In addition to the round or circular perforation 936I, there is also a curved under edge portion 937. In some embodiments, an interconnector for connecting electrodes in an array may form a mechanical connection through the perforation 936I or be welded in the curved under edge portion 937.

In FIGS. 11J-1 and 11J-2, an embodiment of electrode 930 is depicted comprising two anchors 938, one extending from each shorter end of electrode contact 932. Anchors 938 can be embedded or anchored into the silicone or other material of the C-ring portion. For example, the C-ring portion can comprise two channels into which each anchor 938 can slide. The channels and anchors 938 can be relatively configured such that, during positioning and flexing of the C-ring portion, anchors 938 can slide within but remain engaged with the channels. The embodiment of electrode 930 illustrates a folded over (or curved under) anchoring perforation portions 938. For example, the insulating material of the C-ring provided around and/or through the anchors 938 and channel 936J provide improved mechanical coupling, embedding or anchoring of the electrode 930 to the insulating material of the C-ring portion.

The embodiment of FIG. 11K includes two perforations 936K each extending along a longer side of electrode flange 934, curving around two corners and extending partially along each shorter side of electrode flange 934.

Similar to the embodiment of FIG. 11K, the embodiment of FIG. 11L includes two perforations 936L each extending along a shorter side of electrode flange 934, curving around two corners and extending partially along each longer side of electrode flange 934.

The embodiment of FIG. 11M is similar to the embodiment of FIG. 11A except that perforations 936M are circular or round, rather than rounded rectangles or ovals.

The embodiment of FIG. 11N is somewhat similar to the embodiment of FIG. 11I in that it also includes a portion of electrode flange 934 extending from each of four sides, and each portion of electrode flange 934 also comprises a perforation 936N. It further includes a folded over flange portion 934.

The embodiment of FIG. 11O has similarities to the embodiment of FIG. 11I, in that it comprises portions of electrode flange 934 on each shorter end of electrode contact 932, and these portions each include a round or circular perforation 936O. In contrast with the embodiment of FIG. 11I, however, the portions of electrode flange 934 on each shorter end of electrode contact 932 are approximately perpendicular to the electrode contact at each end, rather than continuing in the same plane therefrom.

A spring or a micro-coil (or any other interconnection) connecting the electrode and the lead conductor or between electrodes could be provided through the substantially round perforation 936O. In this way, the stress on the welding is reduced as the connection is already partially held in place by its placement in relation to the round perforation 936O.

Still other configurations of electrode contact 932, electrode flange 934, and electrode perforations 936 are possible in other embodiments. For example, in various embodiments some or all of perforations 936 may not extend completely through electrode flange 936. In other words, perforations 936 instead may be considered to be recesses. Additionally, other shapes, sizes, positions, arrangements, features, dimensions and other characteristics of any of electrode contact 932, electrode flange 934, and electrode perforations 936 can be implemented in other embodiments and may be selected according to a desired application of a particular neural interface in which electrode 930 is implemented.

As in other embodiments of neural interfaces depicted and discussed herein, and even if not explicitly depicted in the drawings, neural interface 900 may also include at least one attribution that may be positioned on an outer surface of neural interface 900, such as on spinal portion 902. The attributions may include one or more openings or eyelets for receiving a stylet (made of tungsten or similar material) or a connector such as a suture thread to releasably connect to the deployment tab, for instance, in order to enable the C-ring portions to be manipulated or to deploy neural interface 900. The attributions may be configured to enable a deployment tool to grip, manipulate and deploy neural interface 900. In one embodiment, the attributions may be placed sufficiently near the open ends of at least one of the C-ring portions 904, 906, 908 to enable the deployment tool to grip the attributions and simultaneously open C-ring portion 906 relative to C-ring portions 904 and 908. This enables positioning of neural interface 900 around a target vessel. Once neural interface 900 has been positioned around the target vessel, the deployment tool (via physician manipulation) can carefully release the attributions so that C-ring portions 904, 906, 908 can softly self-size to the target vessel. The configuration of neural interface 900 may enable the neural interface to be positioned in a single pass around a nerve or vessel with reduced manipulation of the nerve or vessel and a reduction in tissue dissection around the area of the nerve or vessel where the interface is positioned.

Like neural interfaces 100, 200, 300 and 400, neural interface 900 also can be self-sizing, in that C-ring portions 904, 906, 908 in particular are formed of flexible materials and arranged with alternating open ends to provide for easy manipulation for deployment and, when released, return to a predetermined shape without a strong elastic snap or spring force. This enables neural interface 900 to accommodate anatomical variability of the intervention site and target vessel while still providing good electrical contact between the electrode arrays and the surface of the nerve or vessel, thereby improving the efficacy of neural interface 900. The flexible material of C-ring portions 904, 906, 908 may remain compliant even when self-sized to a nerve or vessel. This may help to prevent neural interface 900 from compressing a nerve or vessel and causing reduced blood flow and otherwise constricting nerve fiber. This also may better accommodate radial expansion of the nerve or vessel as a result of post-positioning edema or swelling and may accommodate the pulsatile behavior of intervention sites such as arteries.

The naturally open structure of C-rings 904, 906, 908 portion of neural interface 900 may reduce coverage of the nerve or vessel periphery in a way that promotes more normal fluid and nutrient exchange with the intervention site and surrounding tissue. This may also help to reduce growth of connective tissue into neural interface 900. The open structure of neural interface 900 is configured such that no end portion or center portion forms a closed circumscribed circular arc around the target vessel at any point along a length of the target vessel. In other words, no closed circle covering 360 degrees of an orthogonal portion of the target vessel's length is formed by the structure. However, a tip of the arm may be in contact with the spine of the cuff. In other words, whilst a full coverage of the target vessel may be provided but not via a closed circle. This open unrestricted trench may serve so that the target vessel may pulsate without constriction and that an initially swollen target vessel can return to a normal state over time without constriction when or if it is swollen and without losing electrode-to-target vessel contact when the target vessel is in its normal state.

As previously mentioned, the electrodes (e.g., of electrode array 912 in FIG. 9A or as depicted in and discussed with respect to any of the figures herein) can be embedded within the material of the cuff of the neural interface. Examples of embedded electrodes are depicted in FIGS. 12A and 12B. In FIG. 12A, each electrode 1212 is at least somewhat similar to the embodiments depicted in FIGS. 11A-11N. In FIG. 12B, each electrode 1212 is at least somewhat similar to the embodiment depicted in FIG. 11O. The level or degree of embeddedness between the electrodes in FIG. 12A and those in FIG. 12B can be seen in particular in the partially transparent view of the neural interface of FIG. 12B.

Additionally, different electrode embodiments can provide different degrees of coverage with each cuff of the neural interface. This can be seen in FIG. 12C, in which the electrodes of FIGS. 12A (shown on the left) each provide a greater degree (i.e., percentage) of coverage of the internal cuff surface than the electrodes of FIG. 12B (shown on the right). One or the other may be advantageous or preferred in some applications or embodiments.

For example, and referring also to FIGS. 13A, 13B, and 13C, different electrode embodiments can be used in different sizes of neural interfaces (or cuffs of neural interfaces). Figures 13A, 13B, and 13C depict smaller, medium, and larger cuff diameters, respectively. Additionally, and consistent with discussion herein above, as the cuff diameter increases the thickness of the cuff arm also increases. Thus, the example embodiments depicted are as follows:

| **Embodiment** | **Internal Cuff Diameter** | **Cuff Arm Thickness** | **Number of Electrodes** |
|---|---|---|---|
| FIG. 13A | 6.5mm | 1.0mm | 4 |
| FIG. 13B | 7.7mm | 1.2mm | 5 |
| FIG. 13C | 9.3mm | 1.4mm | 6 |

The variations in internal diameter, arm thickness and number of electrodes can be attributed to maintaining desired contact and tension of the cuff arms and electrode contact area as the diameter (and therefore the length) of the cuff arm decreases or increases. Thus, inner diameters of the neural interface devices can differ while a total electrode area of each neural interface device is substantially equal. Additionally, an electrode of a larger inner diameter neural interface device can comprise a smaller width and a larger length than an electrode of a smaller inner diameter neural interface device.

In order to define the different size and shape of the electrode, the neural interface size is considered. That is, the electrode shape and size can be determined by the relevant neural interface diameter. In other embodiments or applications, different factors may be considered when sizing the cuff and determining a number of electrodes. In some embodiments, each arm of the cuff can be the same as the others, while in other embodiments there may be differences in size or electrode number or configuration between arms of the same cuff.

In some aspects, the inter-electrode coils (such as coils 922 in FIG. 9A) may be replaced with a continuous coil or any other continuous interconnection. Continuous coil embodiments may advantageously reduce the mechanical load on the weld making the weld more reliable and reduce the impact of any one weld failure. For example, FIG. 14A illustrates another embodiment of a neural interface 1400 in accordance with the present disclosure. Neural interface 1400 may be similar to neural interfaces 100, 200, 300, 400, 900 discussed herein above unless otherwise mentioned herein. For example, neural interface 1400 may be formed of the same or a similar flexible substrate of material (i.e., silicone) and share other features.

Neural interface 1400 comprises a spinal portion 1402, a first C-ring portion 1404, a second C-ring portion 1406, and a third C-ring portion 1408. Spinal portion 1402 comprises a first end 1401 coupled to a lead body 1417 comprising a conductor 1418, and a second end 1403 at least partially coupled to first C-ring portion 1404. At least a portion of conductor 1418 extends from lead body 1417 and within spinal portion 1402 from first end 1401 towards second end 1403, terminating in a connection to first C-ring portion 1404. At an opposite end, lead body 1417 and conductor 1418 are connectable via a connector to an implantable pulse generator (not depicted).

First end 1401 of spinal portion 1402 defines a tapered portion that tapers from a maximum circumference to a minimum circumference. In the embodiment of FIG. 14A, the maximum circumference occurs at a point proximate the C-ring portions 1404, 1406,1408, in particular where spinal portion 1402 is at least partially coupled to third C-ring portion 1408. The minimum circumference occurs where spinal portion 1402 terminates along lead body 1417. The length and dimensions of the tapered portion of first end 1401 provide the benefits of reducing the stiffness gradient in transitioning from the relatively stiffer spinal portion 1402 to the relatively more flexible lead body 1417.

Second end 1403 presents an angled, blunted, or rounded-off surface, in that spinal portion 1402 extends to an outer edge of first C-ring portion 1404 at a bottom or lower side (with respect to the orientation of FIG. 14A on the page) but terminates further back on a top or upper side. In other words, spinal portion 1402 has a substantially circular cross-section, and a plane parallel to the circular cross-section is at an angle of greater than 0 degrees and less than 90 degrees with respect to the angled surface of the second end. This surface can be substantially flat, curved, or contain both flat and curved portions.

Between first end 1401 and second end 1403, one end of each of first C-ring portion 1404, second C-ring portion 1406, and third C-ring portion 1408 is coupled to spinal portion 1402.

In some embodiments, each C-ring portion 1404, 1406, 1408 may have a very low helix angle, i.e., pitch, relative to the spinal portion 1402. The helix angle may be between approximately 15 and 30 degrees, but may also be less than 15 degrees. Additionally, each C-ring portion 1404, 1406, 1408 comprises rounded or smooth edges and ends. In other embodiments, each C-ring portion 1404, 1406, 1408 are not helical or have a helix angle, i.e., pitch, relative to the spinal portion 1402, for example as illustrated in Figure 14.

Electrode arrays 1412 may be connected by a continuous coil 1422. Use of a continuous coil 1422 may contribute to greater durability of the overall neural interface 1400 by reducing the number of interconnection points required within the electrode arrays 1412. Using a continuous interconnector, such as continuous coil 1422 reduces interconnection points such as the weld joints compared to some of the embodiments described above.

In some embodiments, continuous coil 1422 can provide greater potential contact area between coil 1422 and electrode arrays 1412 than would be present in other embodiments. This greater contact can help achieve stronger electrical and mechanical connections between coil 1422 and electrode arrays 1412. For instance, since continuous coil 1422 extends along the full length of electrode array 1412, continuous coil 1422 may be welded to electrode array 1412 at multiple points. Multiple individual turns of continuous coil 1422 may be welded to the electrode array, such as with the weld orientation shown in FIG. 9F-2.

FIG. 14A depicts a continuous coil 1422 that is attached to the electrode array 1412 via a bushing, or a sleeve, such as crimped bushing 1430. Use of a bushing such as crimped bushing 1430 helps achieve both mechanical and electrical connection via a single or multiple weld points at the bushing rather than welding the continuous coil 1422 directly to the electrode array 1412, as discussed above. FIG. 14B shows how use of bushing 1430 to connect the coil to array 1412 enable multiple weld points 1434 to strengthen the connection and reduce the chance of any single weld failure from leading to a loss of connection between the coil and the array. The material of the bushing may generally be a conductive material, such as platinum. The material of the bushing may be selected according to the material selection of the continuous coil, the electrode array, and the C-ring, such as to promote good electrical conductivity and a stable weldment. The crimped bushing 1430 fits around the continuous coil by an interference fit, thereby providing electrical and mechanical coupling between the continuous coil 1422 and at least one electrode of the electrode array 1412.

In embodiments, bushing 1430 may be curved to match the curvature of at least one electrode of the electrode array 1412, thus increasing the contact between the bushing and the array and thus providing for a greater points of contact that can be good candidates for welding points between the bushing and the array. Thus, more desirable contact points may be chosen for welding or the number of weld may be increased as required, which may strength the connection between the bushing and the array (and ultimately between the coil and the electrode). A match in curvature between bushing and array can also reduce the mechanical stress placed on the welds connecting the bushing and the array during use. In embodiments, the bushing may be crimped to close the tunnel gaps and hold the wire by interference fit. It is noted that whilst welding is referred to, other forms of connection may be used between the bushing and the array, including but not limited to soldering, crimping, brazing, wiring, or otherwise fastening to create electrical and mechanical connection.

Use of continuous coil 1422 to connect electrode arrays 1412 enables the electrodes in one of the electrode arrays 1412 to be electrically connected in parallel. Thus, loss of a connection between the coil 1422 and any one electrode will not interrupt the supply of power to any other electrodes, even if the connection is lost to an electrode which is "upstream" (closer to alpha connection 1432 between the conductor 1418 and the continuous coil 1422, or simply closer to the conductor 1418) of the remaining connected electrodes. For instance, if bushing 1430a lost its connection to electrode 1412a, electrode 1412a may not be connected to any powering means to provide stimulate or block the target. However, since continuous coil 1422 carries power from the conductor 1418 to electrodes 1412b-d independent of the connection between the coil 1422 and first electrode 1412a, each of electrode array 1412a-d remains operable independent of the condition of any electrode and continuous coil connection in the same C-ring 1404. In this particular embodiment the continuous coil 1422 is connected to the conductor 1418 via alpha helix 1432. In other embodiments, the continuous coil 1422 may be connected directly to the conductor 1418. For example, a tip of the continuous coil may form the alpha helix 1432.

Other embodiments are envisioned which may also achieve the advantages of the continuous coil example in FIG. 14A. In FIG. 14C, neural interface 1440 uses extended jumper coils 1442 to provide improved strain relief and better decoupling forces on the weld joint (for example compared to the smaller inter-electrode coils 922 of FIG. 9A). As shown in the insert of FIG. 14C, alpha helix 1432 includes an alpha-weld hull part coupled to a jumper coil, which is in turn laser welded to an electrode. In FIG. 14C, the jumper coil is welded to the edge of the electrode, but the position of the weld on the electrode can be varied in different versions and embodiments. The position of the welds for attachments between crimps or coils coupling one electrode to another can also differ between embodiments, as shown in more detail in FIG. 14D. In embodiments, the weld/joining position may generally be in a central portion of the electrode. In a preferred embodiment, the ratio of the gap between the electrode to the interconnector (e.g. interconnecting micro-coil or interconnecting coil) is around 1:3 (i.e. the interconnector is about 3x longer compared to the gap between electrodes) or it can be about 1:1. In still further embodiments, the ratio of gap length to the interconnector could be about 1:2.

In FIG. 14D, neural interface 1450 uses continuous jumper coil 1452, which differs from continuous coil 1422 in FIG. 14A by not directly connecting to the conductor 1408 (or the alpha helix 1432). Continuous jumper coil 1452 may be welded directly to electrode arrays 1412 or otherwise attached, such as with crimp bushing 1454. In FIG. 14E, neural interface 1460 uses continuous stranded cable 1462 to connect electrode arrays 1412. Bushing or sleeve 1464 (which may also be crimped for interference fit, or connected by other means) may be used to connect continuous cable 1462 to the individual electrodes in the electrode array 1412. In a similar manner to the embodiments with continuous jumper coils discussed above, the continuous stranded cable 1462 also provides parallel connection of between the electrodes.

In effect, the continuous jumper coil 1452 coupled to each of the electrodes in an array 1412 makes a parallel electrical connection. The connection to the conductor 1408 is provided to each of the electrode arrays 1412 such that even if any one of the connections is lost, the other electrodes 1412 remain powered.

Electrode coils 1422 can be configured to provide electrical couplings between adjacent electrodes of electrode arrays 1412 while also providing desired flexibility and high flex fatigue performance. Conformity or flexibility of C-ring portions 1404 and 1408 can be retained or enhanced by adjusting the diameter and pitch of electrode coils 1422. In example embodiments, a coil pitch of electrode coils 1422 can be in a range of 0.05 mm to 0.3 mm, such as in a range of 0.10 mm to 0.25 mm, for example 0.10 mm, 0.15 mm, or 0.23 mm. A wire diameter of electrode coils 1422 can be in a range of 0.05 mm to 0.10 mm, such as a range of 0.07 mm to 0.09 mm, for example 0.076 mm or 0.081 mm, in various example embodiments. A coil diameter of electrode coils 1422 can be in a range of 0.2 mm to 0.6 mm, such as in a range of 0.3 mm to 0.5 mm, for example 0.38 mm, 0.43 mm, or 0.46 mm. In various embodiments, these dimensions can be selected from example ranges according to a determined relationship between any of these dimensions or other dimensions or characteristics of the electrodes, the C-ring portion, or the overall neural interface.

Crimped bushing 1430 may be crimped to a final size, according to the size of continuous coil 1422, or a final force. Crimping may increase the electrical contact between the coil and the bushing and in embodiments are designed to be tight enough upon crimping (i.e., small enough in cross-section) to promote electrical and mechanical contact between the coil and the bushing. The compressive force or minimum final size of the crimp may be limited, in embodiments, in order to prevent deformation (or extent of deformation) of the coil.

In FIG.14E, another embodiment for providing parallel electrical connection of electrodes with reduced connection interconnections is illustrated. In this embodiment, the electrode comprises pre-formed or inbuilt sleeves (or crimps or tunnels) for accommodating a continuous interconnector (for example a wire, strip or coil). These inbuilt sleeves (or crimps or tunnels) are provided on a rear face of the electrode (the electrode may comprise a target facing surface and a rear side surface). Once the interconnector for connecting the electrodes in an array is threaded through the inbuilt sleeves, a mechanical and electrical coupling of the sleeve and the interconnector may be achieved by crimping the sleeve, by welding or at least partly filling the sleeve with conducting material. The size of the sleeve may be determined by the thickness of the interconnector.

In the embodiment depicted in FIG. 14A, there are no electrodes arranged on second C-ring portion 1406 and the same number and arrangement of electrodes in electrode arrays 1412 on first and third C-ring portions 1404 and 1408. In other embodiments, the number and arrangement of electrodes or electrode arrays 1412 on any individual C-ring portion 1404, 1406, 1408 can vary, with more or fewer electrode arrays 1412 used overall or more or fewer electrodes arranged on any particular C-ring portion 1404, 1406, 1408. Electrodes may be arranged on one C-ring, some but not all C-ring portions, or all C-ring portions 1404, 1406, 1408. Also as previously noted in relation to other embodiments, the cuff may be used for wireless systems, or the cuff may comprise fewer or more C-ring portions.

In some embodiments, multiple electrodes or an electrode array 1412 on any one C-ring portion, such as are depicted on C-ring portions 1404 and 1408 in FIG. 14A, may be considered to be a single electrode. In other words, in some contexts the embodiment of neural interface 1400 depicted in FIG. 14A comprises two electrodes, one on C-ring portion 1404 and one on C-ring portion 1408, with each electrode comprising multiple (four) electrodes portions.

Like neural interfaces 100, 200, 300, 400 and 900, neural interface 1400 also may be self-sizing, in that C-ring portions 1404, 1406, 1408 in particular are formed of flexible materials and arranged with alternating open ends to provide for easy manipulation for deployment and, when released, return to a predetermined shape without a strong elastic snap or spring force. This enables neural interface 1400 to accommodate anatomical variability of the intervention site and target vessel while still providing good electrical contact between the electrode arrays and the surface of the nerve or vessel. The flexible material of C-ring portions 1404, 1406, 1408 may remain compliant even when self-sized to a nerve or vessel. This may help to prevent neural interface 1400 from compressing a nerve or vessel and causing reduced blood flow and otherwise constricting nerve fiber. This also may better accommodate radial expansion of the nerve or vessel as a result of post-positioning edema or swelling and may accommodate the pulsatile behavior of intervention sites such as arteries. Therefore, disclosed herein is a neural interface comprising a spinal portion having a first end and a second end, a circumference of the first end of the spinal portion tapering from a maximum circumference to a minimum circumference; a lead body coupled to the first end of the spinal portion and comprising a conductor connectable to an implantable pulse generator and extending at least partially into the spinal portion; at least three C-ring portions each having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion; and at least one electrode arranged on at least one of the at least three C-ring portions and electrically coupled to the conductor.

The neural interface can comprise a plurality of electrodes arranged on at least one of the at least three C-ring portions, wherein adjacent electrodes on the same C-ring portion are electrically coupled by an inter-electrode coil.

Each of the electrodes can comprise an electrode contact on an electrode flange, the electrode flange mechanically coupling the electrode to the C-ring portion and comprising a plurality of perforations.

The spinal portion can have a substantially circular cross-section, and the second end of the spinal portion can have an angled surface such that a plane parallel to the substantially circular cross-section is at an angle of greater than 0 degrees and less than 90 degrees with respect to a plane defined by the angled surface.

The maximum circumference of the first end of the spinal portion can be proximate the at least three C-ring portions, and the minimum circumference of the first end of the spinal portion can occur where the spinal portion terminates on the lead body.

A distance between the maximum circumference to the minimum circumference can be in a range of 2 mm to 5 mm.

The first C-ring portion and the third C-ring portion can be coupled to the spinal portion to move together and relative to the second C-ring portion, and the first C-ring portion and the third C-ring portion can extend from the spinal portion in a direction opposing a direction of the second C-ring portion

At least one of the at least three C-ring portions of the neural interface can have a first thickness at the first end, a second thickness at the second end, and a third thickness at a point between the first end and the second end, wherein the third thickness is greater than the first thickness and the second thickness.

A thickness of the at least one of the at least three C-ring portions of the neural interface can gradually increase between the first end and the point between the first end and the second end.

A thickness of the at least one of the at least three C-ring portions of the neural interface can gradually increase between the second end and the point between the first end and the second end.

The electrode flange of the neural interface can be rectangular with rounded corners.

The plurality of perforations in the electrode flange of the neural interface can comprise at least one perforation on a first side of the electrode flange and at least one perforation on a second opposing side of the electrode flange.

The first side of the electrode flange and the second opposing side of the electrode flange can be longer than a third side and a fourth side of the electrode flange.

Each of the plurality of perforations in the electrode flange of the neural interface can be rectangular with rounded corners.

The neural interface can comprise at least one anchoring tab coupled to the lead body.

The at least one anchoring tab can comprise a coated mesh.

The lead body can comprise at least one undulating section.

A neural interface can be formed by providing a spinal portion having a first end and a second end, a circumference of the first end of the spinal portion tapering from a maximum circumference to a minimum circumference; coupling a lead body to the first end of the spinal portion such that a conductor of the lead body, connectable to an implantable pulse generator, extends at least partially into the spinal portion; coupling at least three C-ring portions to the spinal portion, each of the at least three C-ring portions having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion; and arranging at least one electrode on each of the at least three C-ring portions and electrically coupling the at least one electrode to the conductor.

The neural interface can further comprise a plurality of electrodes on at least one of the at least three C-ring portions, with adjacent electrodes on the same C-ring portion being electrically coupled by an inter-electrode coil.

Each of the electrodes can comprise an electrode contact on an electrode flange, the electrode flange mechanically coupling the electrode to the C-ring portion and comprising a plurality of perforations.

The method can further include forming the spinal portion to have a substantially circular cross-section and the second end of the spinal portion to have an angled surface such that a plane parallel to the substantially circular cross-section is at an angle of greater than 0 degrees and less than 90 degrees with respect to a plane defined by the angled surface.

Forming the neural interface also can include forming at least one of the at least three C-ring portions to have a first thickness at the first end, a second thickness at the second end, and a third thickness at a point between the first end and the second end, wherein the third thickness is greater than the first thickness and the second thickness.

In another embodiment, a neural interface can comprise a spinal portion having a first end and a second end; a lead body coupled to the first end of the spinal portion and comprising a conductor connectable to an implantable pulse generator and extending at least partially into the spinal portion from the first end toward the second end; at least three C-ring portions each having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion, each C-ring portion having an inner diameter and a thickness, with a ratio of the inner diameter to the thickness being in a range of 5:1 to 6:1; and at least one electrode arranged on at least one of the at least three C-ring portions and electrically coupled to the conductor.

In yet another embodiment, a neural interface can comprise a spinal portion having a first end and a second end; a lead body coupled to the first end of the spinal portion and comprising a conductor connectable to an implantable pulse generator and extending at least partially into the spinal portion from the first end toward the second end; at least three C-ring portions each having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion, each C-ring portion being configured such that in use a pressure in a range of about 0 mmHg to about 30 mmHg is applied to a target tissue arranged within the C-ring portions; and at least one electrode arranged on at least one of the at least three C-ring portions and electrically coupled to the conductor.

FIG. 15A shows an example of an electrode assembly of a C-ring portion 1510, according to one embodiment. In the simplest embodiment, the C-ring portion 1510 can include a metal foil strip or ribbon 1512 upon which the electrodes (not shown) are formed. To provide flexibility in the single body electrode array 1512, in some embodiments foil strip 1512 can be cut or formed such that mesh connectors are cut therein to form a flexible foil 1514. Flexible foil 1514 is more flexible than metal foil strip 1512, because the apertures cut therein reduce the cross-sectional area of the foil at regions other than the weld or crimp sites. These reduced cross-sectional areas result in increased flexibility for deployment and a single body (or unitary) array of electrodes without any additional connection between electrodes is provided. In other words, a weldless interconnection (i.e. no welds are used to provide connection between two electrodes within the array) array of electrodes is provided. A longer (along the strip) section with decreased surface area may be provided for increased strain relief. Whilst weldless between the electrodes, in some embodiments welding may be required for connection to the lead body.

As shown in the bottom row of FIG. 15A, certain portions of the surface of the strip 1514 can be stamped to provide active electrode surface 1542 for protruding out of an insulating material of the C-ring portion 1510 (for example as shown in Figure 15D or in Figure 15H). The active electrode surface 1542 can also be laser roughened to provide an electrode with an increased performance. Radial stamping can be used to form the foil into the final desired C-ring shape or provide other desired shaping.

FIGS. 15B-15C show other example embodiments 1520, 1530 of C-ring portions formed likewise to C-ring portion 1510 shown in FIG. 15A. In embodiments, radial stamping or other methods of molding can also provide additional curvature in a z-axis (e.g., as in a ribbon interconnect) in a decreased width section to provide additional strain relief. The area of reduced width in example embodiments 1520, 1530 can increase flexibility and reduce required materials.

FIG. 15D is an example embodiment 1540 which illustrates an example cross-sectional view of the embodiments shown in FIG. 15A-C. As described above with respect to FIGS. 15A-15C, reduced cross-sectional area of the foil makes the final structure more bendable. As shown in FIG. 15D, regions of conductive wire (also referred to as foil or strip) 1542 are exposed to the radially inner edge of the embodiment 1540 while others are internal to that device such that only some portions of the conductive wire 1542, corresponding to electrode regions, are exposed to the target.

FIGS. 15E-15H depict various views of a serpentine portion embodiment 1550, similar to that discussed in above in relation to example C-rings 1520, 1530 of FIGS. 15A-15C, the serpentine portion between electrodes provides another weld-less array of electrodes, wherein the serpentine portion is another form of achieving a decreased cross-sectional surface area for increased flexibility in the portion between electrodes. In embodiments, the serpentine joint 1552 of example embodiment 1550 can be formed as a leaf spring to provide greater flexibility.

The embodiments exemplified in FIGS. 15A - 15H provide an interconnect means without requiring a weld or other non-integral joining means between the electrodes to be used. In other words, it is a unitary embodiment.

FIG. 15I depicts an example embodiment 1560 wherein a platinum ribbon 1562 provides interconnection between electrodes. For example, a platinum ribbon 1562 is welded using a point-to-point 1564 weld across each gap between electrodes to achieve a spring formation with the ribbon. Platinum to platinum welds can provide increased weld strength over mixed material welds. The curve in the ribbon 1562 can provide flexibility, acting as strain relief, when "opening" the cuff for implantation or removal. A completely flat or straight ribbon could direct higher stress or load at a single point, whereas the curve absorbs some of the stress or load that would be applied to the weld otherwise. A straight interconnect may additionally allow for plastic deformation, such as through a permanent "wrinkle" in the material, which could lead to easier breaking. The embodiment illustrated in FIG. 15I may provide a simplified weld configuration compared with some of the coil-based embodiments, due to more surface for welding, e.g., multiple welding or edge welding, depending on the material configuration.

Further as disclosed herein, a system can comprise a neural interface of any of the embodiments disclosed herein above; a lead cap device having a first end and a second end and comprising a body defining an internal cavity that extends from the first end toward the second end, a set screw block arranged in the body such that a setscrew intersects with the internal cavity, and a suture loop coupled to the second end, the lead cap device configured to removably receive a portion of the lead body in the internal cavity and secure the portion of the lead body in the internal cavity by the setscrew; and a deployment tool comprising a tab-style body having a first end and a second end, a first aperture formed in the first end, a second aperture and a third aperture formed in the second end, and a plurality of sets of eyelets formed in the tab-style body between the first end and the second end, the tab-style body further comprising a series of ridges and grooves, and the deployment tool being removably coupleable to the neural interface by the second aperture and the third aperture and by a suture that can be threaded through the first aperture and at least one of the plurality of sets of eyelets.

A neural interface can comprise a lead body comprising a conductor connectable to an implantable pulse generator; and at least one C-ring portion for applying or maintaining a pressure in a range of 0 mmHg to 30 mmHg to a target tissue arranged within the C-ring portion and comprising at least one electrode arranged on the at least one C-ring portion and electrically coupled to the conductor. In the neural interface, the at least one C-ring portion has an inner diameter and a cross-sectional thickness, with a ratio of the inner diameter to the cross-sectional thickness being in a range of 5:1 to 6:1. In embodiments, this ratio can vary substantially. For example, in thin film embodiments a ratio of 40:1 can be achieved, though generally ratios between 10:1 and 3:1 may suffice.

The at least one electrode can comprise an electrode contact on an electrode flange, the electrode flange mechanically coupling the electrode to the C-ring portion and comprising a plurality of perforations. The electrode flange can be rectangular with rounded corners. The electrode flange can comprise a curved under edge. The plurality of perforations can comprise at least one perforation on a first side of the electrode flange and at least one perforation on a second opposing side of the electrode flange. The first side of the electrode flange and the second opposing side of the electrode flange can be longer than a third side and a fourth side of the electrode flange. Each of the plurality of perforations can be rectangular with rounded corners. The lead body can comprise at least one strain relieving undulating section.

In embodiments, the neural interface can further comprise a spinal portion having a first end and a second end, a circumference of the first end of the spinal portion tapering from a maximum circumference to a minimum circumference, with the lead body being coupled to the first end of the spinal portion and extending at least partially into the spinal portion. The spinal portion can be a substantially circular cross-section, and the second end of the spinal portion can have an angled surface such that a plane parallel to the substantially circular cross-section is at an angle of greater than 0 degrees and less than 90 degrees with respect to a plane defined by the angled surface. The maximum circumference of the first end of the spinal portion can be proximate the at least three C-ring portions, and the minimum circumference of the first end of the spinal portion can occur where the spinal portion terminates on the lead body. A distance between the maximum circumference and the minimum circumference is in a range of 2 mm to 5 mm.

In embodiments, the neural interface can further comprise at least two further C-ring portions, each C-ring portion having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion. The first C-ring portion and the third C-ring portion can be coupled to the spinal portion to move together and relative to the second C-ring portion, and the first C-ring portion and the third C-ring portion can extend from the spinal portion in a direction opposing a direction of the second C-ring portion. At least one of the at least three C-ring portions has a first thickness at the first end, a second thickness at the second end, and a third thickness at a point between the first end and the second end, and the third thickness can be greater than the first thickness and the second thickness. A thickness of the at least one of the at least three C-ring portions gradually increases between the first end and the point between the first end and the second end. A thickness of the at least one of the at least three C-ring portions gradually increases between the second end and the point between the first end and the second end.

The neural interface can further comprise a plurality of electrodes arranged on at least one of the at least three C-ring portions, wherein adjacent electrodes on the same C-ring portion are electrically coupled by an inter-electrode coil.

The neural interface can further comprise at least one anchoring tab coupled to the lead body. The at least one anchoring tab can comprise a coated mesh.

The C-ring portion can be provided at a first end of the lead body and an IPG connector can be provided at a second of the lead body, further wherein the anchoring tab can be provided between the first end and the second end of the lead body.

The anchoring tab can be provided between the first end of the lead body and a middle section of the lead body situated half-way between the first end and the second end of the lead body, further wherein a ratio of a distance between the first end of the lead body and the anchoring tab and a distance between the second end of the lead body and the anchoring tab can be between 1: 1 and 1:50, optionally 1:2, 1:3, 1:4 or 1:5. The anchoring tab can be moveable along the lead body.

The lead body can comprise increased flexibility in a portion closer to the C-ring portion compared to a portion of the lead body further away from the C-ring portion.

In an embodiment, a system comprises the neural interface according to any embodiment, configuration, or combination herein above; and a deployment tool removably coupleable to the neural interface for deployment of the neural interface. The deployment tool can comprise a first area configured to be positioned near the neural interface; and a connector, for releasably coupling the first area to the neural interface, anchored to the first area. The deployment tool can comprise a planar shape or a triangular shape.

In embodiments, the deployment tool can further comprise a second area; and a central area between the first area and the second area. The first area can be wider than the second area.

A cut through the deployment tool can cut through the connector and release the coupling between the deployment tool and the neural interface for at least the first area to move away from the neural interface device.

The deployment tool can further comprise at least one passage extending from the first area to the second area through the central area, each passage including a first opening in the first area and a second opening in the second area.

The connector can be a suture thread for passing through the at least one passage from the second opening to the first opening and for holding the first area near the implantable device, and anchored to the first area.

The deployment tool can further comprise a cuttable portion extending across the at least one passage and configured to release at least one portion of the connector within the at least one passage when the cuttable portion is cut through, wherein the release of the at least one portion of the suture thread enables the first area to move away from the implantable device.

The connector can include a first portion that passes through the at least one passage from the second opening to the first opening, wherein the connector includes a second portion that is removably attached to the implantable device, wherein the connector includes a third portion that passes through the at least one passage from the first opening to the second opening, and wherein the first portion is connected to the second portion and the second portion is connected to the third portion.

The at least one passage can include a first passage and a second passage, wherein the first portion passes through the first passage, the third portion passes through the second passage.

At least the first area and the second area can include rounded edges.

The cuttable portion can be a depressed area in the central area that extends across at least the first passage and the second passage. The depressed area in the central area can extend only across a portion of width of the central area so that at least a portion of the central area is not cut into two pieces when the depressed area is cut through to release the connector. The depressed area can extend across a whole width of the central area so that the central area is cut into two pieces when the depressed area is cut through to release the connector. At least the central area can include a series of alternating lateral ridges and lateral valleys that extend across a width of the central area, for providing longitudinal flexibility that enables the deployment tool to be rolled up while providing lateral stiffness when the deployment tool is unrolled. The first area and the second area include the alternating lateral ridges and lateral valleys that extend across a width of the first area and a width of the second area. The at least one passage can be formed by a tunnel through each lateral ridge and a tube across each lateral valley. The cuttable portion can be a lateral valley. The connector can be anchored to the first area by being molded into the first area. The connector can be anchored to the first area by adhesive. The first area, the second area and the central area can be molded from silicone. At least the second area can be tapered toward the second opening. The tapered second area can include a gripping point for manipulation. The gripping point includes an opening.

The deployment tool can include a first surface and a second surface opposite the first surface, the first surface providing an indication of the location of the cuttable portion, the second surface including a plurality of longitudinal grooves along a length of the deployment tool for reduced contact.

At least the second area and the central area can be tapered, wherein a first portion of the plurality of longitudinal grooves can extend from the first area to the second area through the central area and a second portion of the plurality of longitudinal grooves can extend from the first area to the central area. The second area can taper in its thickness from an edge of the second area towards the central area. The thickness can increase from the edge of the second area towards the central area. The second area can comprise a rounded edge.

The neural interface can be a cuff comprising a spine and at least two curved arms extending from the spine and comprising electrodes, wherein each open end of the curved arm is removably coupled to the deployment tool.

The neural interface can comprise a first arm for being moved in a first direction and one or more second arms for being moved in a second direction substantially opposite the first direction, and the second portion of the connector can be removably attached to the one or more second arms. The one or more second arms can include two arms positioned on opposite sides of the first arm, one arm among the two arms aligned with the first opening of the first passage and the other arm among the two arms aligned with the first opening of the second passage. The one or more second arms can include a first eyelet and the other arm includes a second eyelet, and the second portion of the connector can be removably attached to the cuff by passing through the first eyelet and the second eyelet so as to hold the first area near the cuff until at least one of the first portion or the third portion is cut through at the cuttable portion so that the second portion of the connector can be pulled away from the cuff. A thickness of the central area of the tab can be equal or larger than a thickness of the neural interface. The one or more second arms can have an arm height in a direction perpendicular to both a width and length of the tab, wherein the central area has a height that runs substantially parallel to the arm height, and wherein the height of the central area is greater than the arm height. A width of the first area of the tab is equal or larger than a width of the neural interface.

The cuff can have a width measured from an outer side of the one arm to an outer side of the other arm and that runs substantially parallel to the width of the first area, and wherein the width of the first area is greater than a width of the cuff.

The deployment tool can be configurable as a measurement tool for measuring a fit of the neural interface to a target. A measurement of a fit can be determined based on a distance between the ridges or grooves or valleys of the deployment tool. A measurement of a fit can be determined based on a distance between a first portion of the deployment tool and a second portion of the deployment tool.

The system can further comprise a lead cap device having a first end and a second end and comprising a body defining an internal cavity that extends from the first end toward the second end, a set screw block arranged in the body such that a setscrew intersects with the internal cavity, and a suture loop coupled to the second end, the lead cap device configured to removably receive a portion of the lead body in the internal cavity and secure the portion of the lead body in the internal cavity by the setscrew. An IPG connector portion of the lead body can be removably received in the internal cavity of the lead cap device.

A system can comprise a set comprising a plurality of neural interface devices according to any embodiment discussed or disclosed herein, wherein inner diameters of the neural interface devices differ whilst a total electrode area of each neural interface device is substantially equal. An electrode of a larger inner diameter neural interface device can comprise a smaller width and a larger length than an electrode of a smaller inner diameter neural interface device.

In some embodiments, neural interface includes a spinal portion; a conductor at least partially arranged in the spinal portion; at least three C-ring portions each having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion; and at least one electrode array arranged on at least one of the at least three C-ring portions and electrically coupled to the conductor, each of the at least one electrode arrays comprising one or more electrodes with adjacent electrodes in a respective electrode array being electrically coupled by an inter-electrode coil, each electrode comprising an electrode contact on an electrode flange, the electrode flange mechanically coupling the electrode to the C-ring portion and comprising a plurality of perforations.

In an embodiment, a neural interface comprises a spinal portion having a first end and a second end, a circumference of the first end of the spinal portion tapering from a maximum circumference to a minimum circumference; a lead body coupled to the first end of the spinal portion and comprising a conductor connectable to an implantable pulse generator and extending at least partially into the spinal portion; at least three C-ring portions each having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion; and at least one electrode arranged on at least one of the at least three C-ring portions and electrically coupled to the conductor.

In an embodiment, a method of forming a neural interface comprises providing a spinal portion having a first end and a second end, a circumference of the first end of the spinal portion tapering from a maximum circumference to a minimum circumference; coupling a lead body to the first end of the spinal portion such that a conductor of the lead body, connectable to an implantable pulse generator, extends at least partially into the spinal portion; coupling at least three C-ring portions to the spinal portion, each of the at least three C-ring portions having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion; and arranging at least one electrode on each of the at least three C-ring portions and electrically coupling the at least one electrode to the conductor.

In another embodiment, a neural interface can comprise a spinal portion having a first end and a second end; a lead body coupled to the first end of the spinal portion and comprising a conductor connectable to an implantable pulse generator and extending at least partially into the spinal portion from the first end toward the second end; at least three C-ring portions each having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion, each C-ring portion having an inner diameter and a thickness, with a ratio of the inner diameter to the thickness being in a range of 5:1 to 6:1; and at least one electrode arranged on at least one of the at least three C-ring portions and electrically coupled to the conductor.

In yet another embodiment, a neural interface can comprise a spinal portion having a first end and a second end; a lead body coupled to the first end of the spinal portion and comprising a conductor connectable to an implantable pulse generator and extending at least partially into the spinal portion from the first end toward the second end; at least three C-ring portions each having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion, each C-ring portion being configured such that in use a pressure in a range of about 0 mmHg to about 30 mmHg is applied to a target tissue arranged within the C-ring portions; and at least one electrode arranged on at least one of the at least three C-ring portions and electrically coupled to the conductor.

In a further embodiment, a system can comprise a neural interface of any of the embodiments disclosed herein; a lead cap device having a first end and a second end and comprising a body defining an internal cavity that extends from the first end toward the second end, a set screw block arranged in the body such that a setscrew intersects with the internal cavity, and a suture loop coupled to the second end, the lead cap device configured to removably receive a portion of the lead body in the internal cavity and secure the portion of the lead body in the internal cavity by the setscrew; and a deployment tool comprising a tab-style body having a first end and a second end, a first aperture formed in the first end, a second aperture and a third aperture formed in the second end, and a plurality of sets of eyelets formed in the tab-style body between the first end and the second end, the tab-style body further comprising a series of ridges and grooves, and the deployment tool being removably coupleable to the neural interface by a suture that can be threaded through the first aperture and at least one of the plurality of sets of eyelets.

In addition to or as an alternative to the above, examples consistent with the present teachings are set out in the following numbered clauses.

Features and components of different embodiments discussed herein can be combined in other embodiments. Additionally, features and components discussed herein with respect to particular embodiments or types of neural interfaces or devices can be used with other devices, including other types of electrodes and leads. For example, lead features designed to reduce strain can be used in a variety of other types of devices in which lead strain may be an issue. In another example, component configurations for laser-welding can have applicability in other types of devices and structures. Those skilled in the art will recognize how still other features and components discussed herein may be used with other devices and systems as well as in other applications and methods. In this way particular effects can be designed and achieved in order to meet particular desires or needs in the industry. Dimensions given in the description or drawings are examples and can vary, independently or in combination, in other embodiments. Ranges or dimensions disclosed as being "about" or "approximately" a value can vary by plus/minus 5 percent of the value.

Various embodiments of systems, devices, and methods have been described herein. These embodiments are given only by way of example and are not intended to limit the scope of the claimed inventions. It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the claimed inventions.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

Although a dependent claim may refer in the claims to a specific combination with one or more other claims, other embodiments can also include a combination of the dependent claim with the subject matter of each other dependent claim or a combination of one or more features with other dependent or independent claims. Such combinations are proposed herein unless it is stated that a specific combination is not intended.

Applicant incorporates by reference the contents of the previously-filed PCT application published as WO 2019/020986. In particular, the electrode described herein may be replaced with the coil electrode described in this application. Any incorporation by reference of documents above is limited such that no subject matter is incorporated that is contrary to the explicit disclosure herein. Any incorporation by reference of documents above is further limited such that no claims included in the documents are incorporated by reference herein. Any incorporation by reference of documents above is yet further limited such that any definitions provided in the documents are not incorporated by reference herein unless expressly included herein.

For purposes of interpreting the claims, it is expressly intended that the provisions of 35 U.S.C. § 112(f) are not to be invoked unless the specific terms "means for" or "step for" are recited in a claim.

### EMBODIMENTS

1. A neural interface comprising:
   at least one C-ring portion for applying a radial pressure in a range of 1 mmHg to 30 mmHg to a target tissue arranged within the C-ring portion and comprising at least one electrode arranged on the at least one C-ring portion.
2. The neural interface of embodiment 1, further comprising a lead body comprising a conductor connectable to an implantable pulse generator, wherein the at least one electrode is electrically coupled to the conductor.
3. The neural interface of embodiment 1, wherein the C-ring portion applies a radial pressure based upon one or more of the group comprising:
   rigidity of an insulating material that makes up a body of the C-ring portion;
   thickness of an insulating material that makes up the body of the C-ring portion;
   rigidity of the at least one electrode;
   a size and shape of the at least one electrode;
   a quantity of the electrode;
   a proportion of electrode compared to the insulating material of the C-ring portion;
   a gap size between two electrodes of the at least one electrode;
   properties of the interconnect between different electrodes of the at least one electrode;
   thickness of the c-ring material; and
   a diameter of the neural interface.
4. The neural interface of embodiment 1, the at least one C-ring portion having an inner diameter and a cross-sectional thickness, with a ratio of the inner diameter to the cross-sectional thickness being in a range of 5:1 to 6:1.
5. The neural interface of embodiments 1-4, wherein the at least one electrode comprises an electrode contact on an electrode flange, the electrode flange mechanically coupling the electrode to the C-ring portion and comprising a plurality of perforations.
6. The neural interface of any of embodiments 1-5, wherein the electrode flange is rectangular with rounded corners.
7. The neural interface of any of embodiments 1-6, wherein the electrode flange comprises a curved under edge.
8. The neural interface of any of embodiments 1-7, wherein the plurality of perforations comprise at least one perforation on a first side of the electrode flange and at least one perforation on a second opposing side of the electrode flange.
9. The neural interface of any of embodiments 1-8, wherein the first side of the electrode flange and the second opposing side of the electrode flange are longer than a third side and a fourth side of the electrode flange.
10. The neural interface of any of embodiments 1-9, wherein each of the plurality of perforations is rectangular with rounded corners.
11. The neural interface of embodiment 2, wherein the lead body comprises at least one strain relieving undulating section.
12. The neural interface of embodiment 2 or 11, further comprising:
   a spinal portion having a first end and a second end, a circumference of the first end of the spinal portion tapering from a maximum circumference to a minimum circumference;
   the lead body being coupled to the first end of the spinal portion and extending at least partially into the spinal portion.
13. The neural interface of any of embodiments 1-12, wherein the spinal portion has a substantially circular cross-section, and the second end of the spinal portion has an angled surface such that a plane parallel to the substantially circular cross-section is at an angle of greater than 0 degrees and less than 90 degrees with respect to a plane defined by the angled surface.
14. The neural interface of any of embodiments 12 or 13, wherein the maximum circumference of the first end of the spinal portion is proximate the at least three C-ring portions, and the minimum circumference of the first end of the spinal portion occurs where the spinal portion terminates on the lead body.
15. The neural interface of any of embodiments 12-14, wherein a distance between the maximum circumference and the minimum circumference is in a range of 2 mm to 5 mm.
16. The neural interface of any of embodiments 12-15, further comprising:
   at least two further C-ring portions, each C-ring portion having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion.
17. The neural interface of embodiment 16, wherein the first C-ring portion and the third C-ring portion are coupled to the spinal portion to move together and relative to the second C-ring portion, and wherein the first C-ring portion and the third C-ring portion extend from the spinal portion in a direction opposing a direction of the second C-ring portion.
18. The neural interface of any of embodiments 16-17, wherein at least one of the at least three C- ring portions has a first thickness at the first end, a second thickness at the second end, and a third thickness at a point between the first end and the second end, and wherein the third thickness is greater than the first thickness and the second thickness.
19. The neural interface of any of embodiments 16-18, wherein a thickness of the at least one of the at least three C-ring portions gradually increases between the first end and the point between the first end and the second end.
20. The neural interface of any of embodiments 16-19, wherein a thickness of the at least one of the at least three C-ring portions gradually increases between the second end and the point between the first end and the second end.
21. The neural interface of any of embodiments 16-20, further comprising a plurality of electrodes arranged on at least one of the at least three C-ring portions, wherein adjacent electrodes on the same C-ring portion are electrically coupled by an inter-electrode coil.
22. The neural interface of any preceding embodiment, further comprising at least one anchoring tab coupled to the lead body.
23. The neural interface of embodiment 22, wherein the at least one anchoring tab comprises a coated mesh, optionally wherein the mesh is coated with a material that fills the mesh.
24. The neural interface of embodiment 22 or 23, wherein the C-ring portion is provided at a first end of the lead body and a connector to an implantable pulse generator (IPG) is provided at a second of the lead body, further wherein the anchoring tab is provided between the first end and the second end of the lead body.
25. The neural interface of embodiment 24, wherein the anchoring tab is provided between the first end of the lead body and a middle section of the lead body situated half-way between the first end and the second end of the lead body, further wherein a ratio of a distance between the first end of the lead body and the anchoring tab and a distance between the second end of the lead body and the anchoring tab is between 1:1 and 1:50, optionally 1:2, 1:3, 1:4 or 1:5.
26. The neural interface of any of embodiments 22-25, wherein the anchoring tab is moveable along the lead body.
27. The neural interface of any preceding embodiment, wherein the lead body comprises increased flexibility in a portion closer to the C-ring portion compared to a portion of the lead body further away from the C-ring portion.
28. The neural interface of any preceding embodiment, wherein a plurality of electrodes are electrically connected in parallel.
29. The neural interface of any preceding embodiment, wherein the conductor comprises a single continuous coil electrically coupled to a plurality of electrodes on one of the C-ring portions.
30. The neural interface of embodiment 29, wherein the single continuous coil comprises a conductive bushing corresponding to each electrode.
31. The neural interface of embodiment 30, wherein the conductive bushing is crimped for mechanical and electrical connection with the single continuous coil, further wherein each crimped bushing is configured to be welded to each corresponding electrode such that the coil is electrically connected to the electrode.
32. The neural interface of embodiment 28 and 29, wherein the electrode comprises an inbuilt sleeve for accommodating the single continuous coil.
33. A system comprising:
   the neural interface of any preceding embodiment; and
   a deployment tool being removably coupleable to the neural interface for deployment of the neural interface.
34. The system of embodiment 33, wherein the deployment tool is configurable as a measurement tool for measuring a fit of the neural interface to a target.
35. The system of any of embodiments 33 or 34, wherein the deployment tool is configured to function as a blunt dissection tool.
36. The system of any of embodiments 33-35, wherein a thickness of the deployment tool is larger than a thickness of a C-ring portion of the neural interface.
37. The system of any of embodiments 33-35, wherein a width of the deployment tool is larger than a width the neural interface.
38. The system of any of embodiments 33-37, further comprising a lead cap device having a first end and a second end and comprising a body defining an internal cavity that extends from the first end toward the second end, and a suture loop coupled to the second end, the lead cap device configured to removably receive a portion of the lead body in the internal cavity.
39. The system of embodiment 38, wherein an IPG connector portion of the lead body is removably received in the internal cavity of the lead cap device, further wherein the lead cap device comprises a set screw block arranged in the body such that a setscrew intersects with the internal cavity, and is configured to secure the portion of the lead body in the internal cavity by the setscrew.
40. A system comprising a set comprising a plurality of neural interface devices according to any of embodiments 1 to 27, wherein inner diameters of the neural interface devices differ whilst a total electrode area of each neural interface device is substantially equal.
41. The system of embodiment 41, wherein an electrode of a larger inner diameter neural interface device comprises a smaller width and a larger length than an electrode of a smaller inner diameter neural interface device.
42. The system of any of embodiments 33-41, wherein the deployment tool is positioned within the C-ring portion of the neural interface.
43. The system of embodiment 42, wherein the deployment tool is rolled at least partly in the neural interface.

## Claims

1. A system comprising a set comprising a plurality of neural interface devices, each neural interface comprising:
at least one C-ring portion for applying a radial pressure in a range of 1 mmHg to 30 mmHg to a target tissue arranged within the C-ring portion and comprising at least one electrode arranged on the at least one C-ring portion,
wherein inner diameters of the neural interface devices differ whilst a total electrode area of each neural interface device is substantially equal.

2. The system of claim 1, wherein each neural interface further comprises a lead body comprising a conductor connectable to an implantable pulse generator, wherein the at least one electrode is electrically coupled to the conductor,
optionally wherein the lead body comprises at least one strain relieving undulating section.

3. The system of claim 1, wherein the C-ring portion applies a radial pressure based upon one or more of the group comprising:
rigidity of an insulating material that makes up a body of the C-ring portion;
thickness of an insulating material that makes up the body of the C-ring portion;
rigidity of the at least one electrode;
a size and shape of the at least one electrode;
a quantity of the electrode;
a proportion of electrode compared to the insulating material of the C-ring portion;
a gap size between two electrodes of the at least one electrode;
properties of the interconnect between different electrodes of the at least one electrode;
thickness of the c-ring material; and
a diameter of the neural interface.

4. The system of claim 1, the at least one C-ring portion having an inner diameter and a cross-sectional thickness, with a ratio of the inner diameter to the cross-sectional thickness being in a range of 5:1 to 6:1.

5. The system of claims 1-4, wherein the at least one electrode comprises an electrode contact on an electrode flange, the electrode flange mechanically coupling the electrode to the C-ring portion and comprising a plurality of perforations,
optionally wherein the electrode flange is rectangular with rounded corners,
and/or wherein the electrode flange comprises a curved under edge.

6. The system of any of claims 1-5, wherein the plurality of perforations comprise at least one perforation on a first side of the electrode flange and at least one perforation on a second opposing side of the electrode flange,
optionally wherein the first side of the electrode flange and the second opposing side of the electrode flange are longer than a third side and a fourth side of the electrode flange,
and/or wherein each of the plurality of perforations is rectangular with rounded corners.

7. The system of claim 2, wherein each neural interface further comprises:
a spinal portion having a first end and a second end, a circumference of the first end of the spinal portion tapering from a maximum circumference to a minimum circumference;
the lead body being coupled to the first end of the spinal portion and extending at least partially into the spinal portion
optionally wherein the spinal portion has a substantially circular cross-section, and the second end of the spinal portion has an angled surface such that a plane parallel to the substantially circular cross-section is at an angle of greater than 0 degrees and less than 90 degrees with respect to a plane defined by the angled surface,
and/or wherein the maximum circumference of the first end of the spinal portion is proximate the at least one C-ring portion, and the minimum circumference of the first end of the spinal portion occurs where the spinal portion terminates on the lead body.

8. The system of claim 7, wherein a distance between the maximum circumference and the minimum circumference is in a range of 2 mm to 5 mm.

9. The system of any of claims 7 or 8, wherein each neural interface further comprises:
at least two further C-ring portions, each C-ring portion having a first end and a second end, the first end of each C-ring portion being coupled to the spinal portion such that the second ends of a first C-ring portion and a third C-ring portion are on a first side of the spinal portion and the second end of a second C-ring portion, arranged between the first C-ring portion and the third C-ring portion, is on a second opposing side of the spinal portion,
optionally wherein the first C-ring portion and the third C-ring portion are coupled to the spinal portion to move together and relative to the second C-ring portion, and wherein the first C-ring portion and the third C-ring portion extend from the spinal portion in a direction opposing a direction of the second C-ring portion
and/or wherein at least one of the at least three C-ring portions has a first thickness at the first end, a second thickness at the second end, and a third thickness at a point between the first end and the second end, and wherein the third thickness is greater than the first thickness and the second thickness.

10. The system of any of claims 9, wherein a thickness of the at least one of the at least three C-ring portions gradually increases between the first end and the point between the first end and the second end,
and/or wherein a thickness of the at least one of the at least three C-ring portions gradually increases between the second end and the point between the first end and the second end,
and/or wherein each neural interface further comprises a plurality of electrodes arranged on at least one of the at least three C-ring portions, wherein adjacent electrodes on the same C-ring portion are electrically coupled by an inter-electrode coil.

11. The system of any one of claims 2-10, wherein each neural interface further comprises at least one anchoring tab coupled to the lead body
optionally wherein the at least one anchoring tab comprises a coated mesh, optionally wherein the mesh is coated with a material that fills the mesh.

12. The system of claim 11, wherein the C-ring portion is provided at a first end of the lead body and a connector to an implantable pulse generator (IPG) is provided at a second of the lead body, further wherein the anchoring tab is provided between the first end and the second end of the lead body,
optionally wherein the anchoring tab is provided between the first end of the lead body and a middle section of the lead body situated half-way between the first end and the second end of the lead body, further wherein a ratio of a distance between the first end of the lead body and the anchoring tab and a distance between the second end of the lead body and the anchoring tab is between 1: 1 and 1:50, optionally 1:2, 1:3, 1:4 or 1:5.

13. The system of any of claims 11 or 12, wherein the anchoring tab is moveable along the lead body.

14. The system of any one of claims 2-13, wherein the lead body comprises increased flexibility in a portion closer to the C-ring portion compared to a portion of the lead body further away from the C-ring portion.

15. The system of any preceding claim, wherein an electrode of a larger inner diameter neural interface device comprises a smaller width and a larger length than an electrode of a smaller inner diameter neural interface device.
